# EUROPEAN PATENT APPLICATION

(11) **EP 4 502 133 A1**
(43) Date of publication of application: **05.02.2025**
(21) Application number: 22933508.8
(22) Date of filing: 25.03.2022
(51) Int. Cl.: C12M 1/34, C12Q 1/6827, G01N 33/50, G16B 20/20

(54) **INFORMATION PROCESSING DEVICE, INFORMATION PROCESSING METHOD, AND INFORMATION PROCESSING PROGRAM**

(71) Applicant: The University of Tokyo, Bunkyo-ku, Tokyo 113-8654 (JP)
(72) Inventor: YOKOYAMA, Kazuaki, Tokyo 113-8654 (JP)
(74) Representative: Hasegawa, Kan
(86) International application number: PCT/JP2022/014507
(87) International publication number: WO 2023/181370

(57) **Abstract**

The present invention relates to an information processing apparatus, an information processing method, and an information processing program relating to genetic information. Provided are an information processing apparatus that selects a target sequence variation possessed by a subject and having a risk of being harmful, the information processing apparatus including a filtering unit 2 that classifies one or more sequence variations specified by sequencing a nucleic acid included in the subject into different categories according to a degree of risk of being harmful on the basis of one or more classification criteria, and a control unit 3 that classifies a base sequence including a sequence variation of which a category to which the sequence variation is to belong is known into each of the categories according to the degree of risk of being harmful on the basis of at least one of the classification criteria, and compares a classification result with the category to which the sequence variation is to belong, an information processing method, and an information processing program.

## Description

### TECHNICAL FIELD

The present invention relates to an information processing apparatus, an information processing method, and an information processing program for a base sequence.

### BACKGROUND ART

Conventionally, it is widely known that a disease may occur due to a mutation of a base sequence included in genetic information of somatic cells. For example, variations such as single nucleotide polymorphism (SNP) and structural variation (SV) occurring in a gene may cause diseases such as cancer. In recent years, information regarding diseases associated with various base sequence variations in somatic cells has been recorded in a database, and has been widely used (see Non Patent Literature 1).

In addition, in recent years, advances in comprehensive base sequence interpretation technology (for example, next generation sequencer (NGS)) have made it possible to interpret the entire genome at an individual level. Therefore, the number of variations detected in a single variation analysis is as enormous as several hundred to several million per specimen, and it is not efficient and realistic to artificially interpret the results of each variation. Therefore, there is a demand for a device that assists in human interpretation of an analysis result.

### Citation List

### Non Patent Literature

Non Patent Literature 1: COSMIC Release v94 is live!, [online], March 28, 2021, [retrieved October 8, 2021], Internet <URL: https://cosmic-blog.sanger.ac.uk/Release-v94/>

### SUMMARY OF INVENTION

### Technical Problem

By interpreting a base sequence of a specimen by using the above database, it is possible to determine whether or not a variation has occurred in the base sequence of the specimen. However, it is not possible to easily determine that a variation present in a base sequence directly affects a disease (for example, driver variations for cancer) with that much information. This is because there are various items to be considered in addition to the variation in order to determine that the variation of the base sequence directly affects the disease. However, analysis has not been performed on how much variation of a base sequence of a specimen may affect the occurrence of diseases in consideration of such various items.

Therefore, the present applicant has filed a patent application for a technology for realizing an analysis apparatus that presents the degree of possibility of variation of a base sequence affecting the occurrence and progression of diseases (see International Application No. PCT/JP2020/037499) .

An object of the present invention is to more accurately present the degree of possibility of variation of a base sequence affecting the occurrence and progression of diseases.

### Solution to Problem

An information processing apparatus according to one aspect of the present invention for solving the above problems is an information processing apparatus that selects a target sequence variation possessed by a subject and having a risk of being harmful on a base sequence, the information processing apparatus including a filtering unit that classifies one or more sequence variations specified by sequencing a nucleic acid included in the subject into different categories according to a degree of risk of being harmful on the basis of one or more classification criteria; and a control unit that classifies a base sequence including a sequence variation of which a category to which the base sequence or the sequence variation is to belong is known into each of the categories according to the degree of risk of being harmful on the basis of at least one of the classification criteria, and compares a classification result with the category to which the base sequence or the sequence variation is to belong.

An information processing method according to an aspect of the present invention is a method of selecting a target sequence variation possessed by a subject and having a risk of being harmful on a base sequence, the method including a filtering step of classifying one or more sequence variations specified by sequencing a nucleic acid included in the subject into different categories according to a degree of risk of being harmful on the basis of one or more classification criteria; and a control step of classifying a base sequence including a sequence variation of which a category to which the base sequence or the sequence variation is to belong is known into each of the categories according to the degree of risk of being harmful on the basis of at least one of the classification criteria, and comparing a classification result with the category to which the base sequence or the sequence variation is to belong.

An information processing program according to an aspect of the present invention is configured to cause a computer to function as the information processing apparatus.

### Advantageous Effects of Invention

According to the present invention, it is possible to more accurately present the degree of possibility of a variation of a base sequence affecting the occurrence and progression of diseases.

### BRIEF DESCRIPTION OF DRAWINGS

Fig. 1 is a block diagram illustrating a configuration example of an information processing apparatus according to an embodiment of the present invention.
Fig. 2 is a functional block diagram illustrating an example of each function of the information processing apparatus according to the embodiment of the present invention.
Fig. 3 is a functional block diagram illustrating an example of a filtering unit of the information processing apparatus according to the embodiment of the present invention.
Fig. 4 is an explanatory diagram illustrating an example of base sequence information input to the information processing apparatus according to the embodiment of the present invention.
Fig. 5 is a functional block diagram illustrating an example of a filter processing unit of the information processing apparatus according to the embodiment of the present invention.
Fig. 6 is an explanatory diagram illustrating an example of output information output by the information processing apparatus according to the embodiment of the present invention.
Fig. 7 is a functional block diagram illustrating an example of a control unit of the information processing apparatus according to the embodiment of the present invention.
Fig. 8 is a flowchart illustrating an operation example of a filtering unit of the information processing apparatus according to the embodiment of the present invention.
Fig. 9 is a flowchart illustrating an operation example of a filter processing unit of the information processing apparatus according to the embodiment of the present invention.
Fig. 10 is a flowchart illustrating an operation example of the control unit and an adjustment unit of the information processing apparatus according to the embodiment of the present invention.
Fig. 11 is a functional block diagram illustrating an example of a filter processing unit of an information processing apparatus according to a second embodiment of the present invention.
Fig. 12 is a flowchart illustrating an operation example of a filter processing unit of the information processing apparatus according to the second embodiment of the present invention.

### DESCRIPTION OF EMBODIMENTS

Hereinafter, an embodiment of the present invention will be described in detail with reference to the accompanying drawings. However, this embodiment is an example, and the present invention is not limited thereto.

A first embodiment of the present invention will be described with reference to the drawings.

An information processing apparatus 1 is an information processing apparatus 1 that selects a target sequence variation having a risk of being harmful on a base sequence, and includes a filtering unit 2 that classifies one or more sequence variations specified by sequencing nucleic acids included in an individual or a specimen (hereinafter, also referred to as a subject) to be subjected to information processing into different categories according to the degree of the risk of being harmful on the basis of one or more classification criteria. In addition, the information processing apparatus 1 includes the control unit 3 that classifies a base sequence including a sequence variation of which a category to which the sequence variation is to belong is known into each of the categories according to the degree of the risk of being harmful on the basis of at least one of classification criteria, and compares the classification result with the category to which the sequence variation is to belong. The filtering unit 2 and the control unit 3 of the information processing apparatus 1 will be described in detail later.

In the present specification, the term "sequence variation" is a variation state of a base sequence including a position and a type of a variation. The sequence variation may be, for example, a single-base variation, or may be a structural variation such as a chromosomal translocation extending over a plurality of genes.

Information including information indicating the sequence variation will be referred to as "base sequence information". The base sequence information may include, as information indicating a sequence variation, information indicating to what kind of base or base sequence a base or a base sequence which should originally be present has varied at a position where the variation has occurred (a position in a chromosome when compared with reference genome information (for example, information indicating the number of a base from one side of a reference base sequence) or the like). The reference genome information is, for example, genome information necessary for NGS interpretation, and examples thereof include GRCh38 (hg38) and GRCh37 (hg19) in humans. In addition, the base sequence information may include information extracted through sequence alignment as information indicating a sequence variation.

Further, the base sequence information may be information acquired by sequencing a base sequence with a next generation sequencer or the like. The base sequence may be a nucleic acid obtained from a subject or may be artificially synthesized. The base sequence information may include, for example, a file in FASTQ format, SAM (Sequence Alignment Map) format, or BAM format as information acquired through sequencing.

The risk of being harmful herein indicates a possibility of occurrence of a disease including cancer. For example, a sequence variation having a risk of being harmful indicates that there is a possibility that a disease such as cancer may occur due to a variation of the base sequence, and a sequence variation having no risk of being harmful is a variation of the base sequence with no such possibility. Note that, the sequence variation to be selected by the information processing apparatus 1 will be particularly referred to as a "target sequence variation".

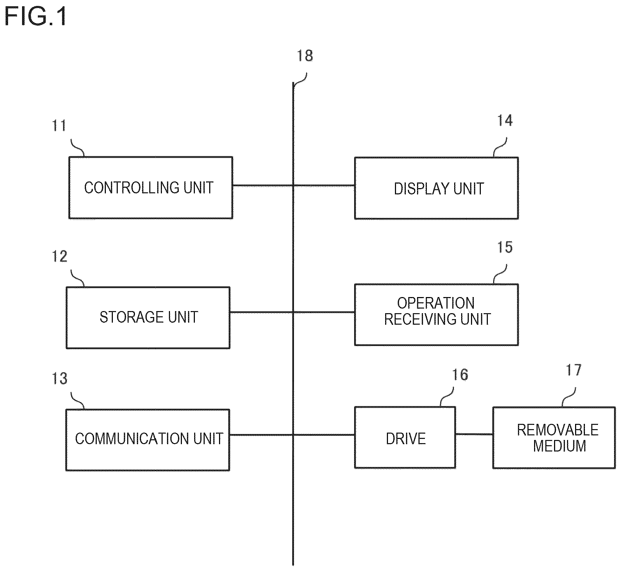

Fig. 1 is a block diagram illustrating a schematic configuration of the information processing apparatus 1. As illustrated in Fig. 1, the information processing apparatus 1 includes a controlling unit 11, a storage unit 12, a communication unit 13, a display unit 14, an operation receiving unit 15, and a drive 16. The constituents are communicatively connected to each other via a bus 18.

The controlling unit 11 includes a CPU (Central Processing Unit), and executes control of each constituent and various types of arithmetic processing according to a program.

The storage unit 12 includes a ROM (Read Only Memory) that stores various programs and various types of data in advance, a RAM (Random Access Memory) that temporarily stores programs and data as a work area, a hard disk that stores various programs and various types of data, and the like.

The communication unit 13 communicates with another device (for example, an information processing apparatus or the like of a terminal from which an analysis result (not illustrated) is viewed) via a network N including the Internet.

The display unit 14 includes a display such as a liquid crystal, a speaker, or the like, and outputs various types of information as an image or a sound.

The operation receiving unit 15 includes a touch sensor, a pointing device such as a mouse, a keyboard, and the like, and receives various operations of a user. Note that the display unit 14 and the operation receiving unit 15 may configure a touch panel by overlapping a touch sensor serving as the operation receiving unit 15 on a display surface serving as the display unit 14. The operation receiving unit 15 may include the drive 16.

A removable medium 17 including a magnetic disk, an optical disc, a magneto-optical disc, a semiconductor memory, or the like may be appropriately mounted on the drive 16. A program read from the removable medium 17 by the drive 16 is installed in the storage unit 12 as necessary.

Furthermore, the removable medium 17 may also store various types of data stored in the storage unit 12, similarly to the storage unit 12.

Various processes can be executed through cooperation of various types of hardware and various types of software of the information processing apparatus 1 in Fig. 1.

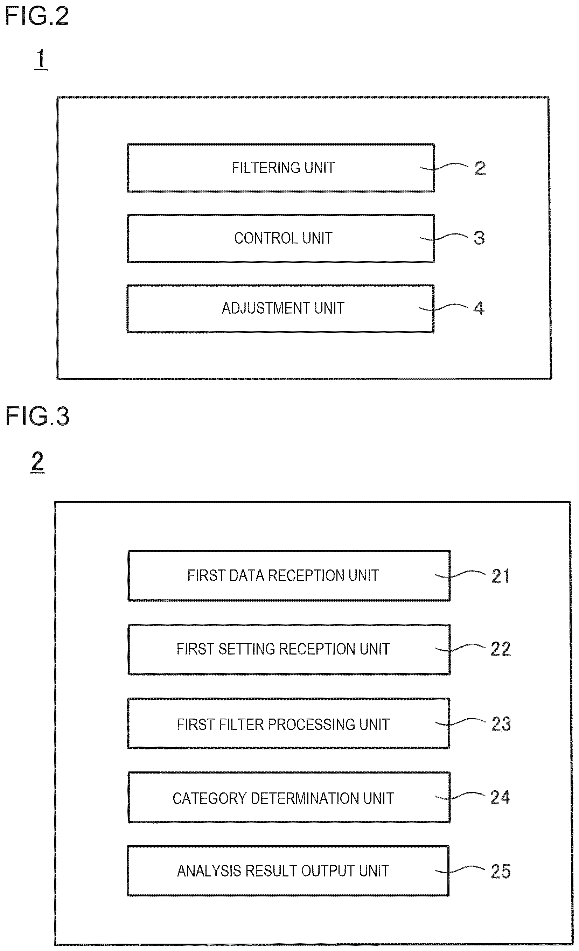

Fig. 2 is a block diagram illustrating a functional configuration of the controlling unit 11 of the information processing apparatus 1 according to the present embodiment. As illustrated in Fig. 2, the controlling unit 11 of the information processing apparatus 1 functions as a filtering unit 2, a control unit 3, and an adjustment unit 4 by reading a program and executing processes.

### <Filtering unit>

The filtering unit 2 classifies one or more sequence variations identified by sequencing a nucleic acid included in a subject into different categories according to the degree of risk of being harmful on the basis of one or more classification criteria. Fig. 3 is a block diagram illustrating an example of a functional configuration for executing various processes related to the filtering unit 2 in the information processing apparatus 1. As illustrated in Fig. 3, the filtering unit 2 functions as a first data reception unit 21, a first setting reception unit 22, a first filter processing unit 23, a category determination unit 24, and an analysis result output unit 25.

### (First data reception unit)

The first data reception unit 21 receives base sequence information including one or more sequence variations specified by sequencing a nucleic acid included in a subject. Hereinafter, the base sequence information received by the first data reception unit 21 will also be referred to as first base sequence information. The first base sequence information may include, in addition to information indicating a sequence variation, an individual to be subjected to information processing, specimen identification information for identifying a specimen obtained from the individual, and the like.

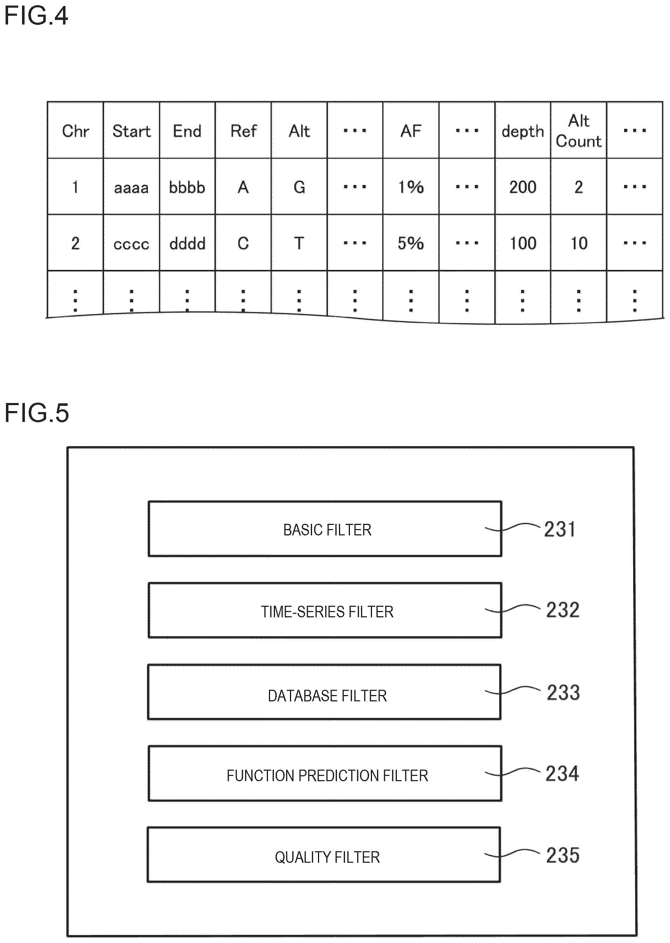

Fig. 4 illustrates a configuration example of the first base sequence information received by the first data reception unit 21 in the information processing apparatus 1 in Fig. 3.

As illustrated in Fig. 4, the first base sequence information is information in which, for each sequence variation (for each row in the drawing), at least a number (Chr) of a chromosome in which a base sequence of the sequence variation is found, a start position (Start), an end position (End), the base sequence (Ref) that should originally be present, the extracted base sequence (Alt) that has varied, and a ratio of the base sequence (allele frequency: AF) that has varied are associated with each other. In addition, the base sequence information received by the second data reception unit 31 that will be described later includes information regarding a category to which the base sequence is to belong, which will be described later.

In the first base sequence information of this example, an index or the like related to quality such as depth and the number of counts of sequence variation (AltCount) is further associated with each sequence variation (each row in the drawing). A length of the base sequence may be "1" (in this case, the base sequence information is information representing any of bases A, T, C, and G).

In addition, the first base sequence information may include information regarding a case or the like of an individual (information such as a disease name, treatment history, and tumor ratio).

In addition, the first data reception unit 21 may receive information (time-series information) regarding base sequences extracted from the same subject at different timings (there may be a plurality of timings). In this case, the first data reception unit 21 may receive time-series inputs of base sequence information to be analyzed.

### (First setting reception unit)

The first setting reception unit 22 receives a setting for analyzing the base sequence information received by the first data reception unit 21. This setting includes, for example, a setting of the type of a filter used for the filter processing unit that will be described later and a setting of a classification criterion in each filter.

### (Filter processing unit)

In the present embodiment, through an operation of the filter processing unit, the degree of risk of being harmful is evaluated on the basis of various types of information that affect the interpretation of an analysis result of a variation of a base sequence. An evaluation result of the degree of risk of being harmful is represented by any of categories MYC1 to MYC4 that will be described later.

Here, the information that affects the interpretation includes (1) additional information regarding the variation obtained at the time of analysis, and (2) information related to the variation listed in documents or databases. Among them, (1) additional information regarding the variation obtained at the time of analysis includes (a) information regarding detection accuracy and reliability (for example, a probability that the variation is not a detection error), (b) an allele frequency of the variation (for example, an index related to the proportion of a cell population having the same variation in the whole), (c) time-series information (for example, whether or not the variation has been repeatedly detected in a specimen at another time point of the same case), and the like.

In addition, (2) the information related to the variation listed in documents or databases includes information indicating whether or not the variation is described as a driver variation of a disease (or how often the variation is described). In a case where there is also registration in the SNP (single nucleotide polymorphism) database, information regarding at what allele frequency a variant allele has been reported as SNP in an ethnic group may be listed in documents or databases. Furthermore, as the function prediction, information indicating whether or not the variation affects a three-dimensional structure or a function of the encoded protein, for example, whether or not it is predicted through experiments or the like as being related to the disease state formation of cancer, and the like may be listed in documents and databases.

### (First filter processing unit)

The first filter processing unit 23 classifies the sequence variation included in the base sequence information received by the first data reception unit 21 into any one of MYC1, MYC2, MYC3, and MYC4, which are categories according to the degree of risk of being harmful, on the basis of one or more predetermined classification criteria. Note that a detailed configuration example of the first filter processing unit 23 will be described later with reference to Fig. 5.

Here, MYC1 and MYC2 are categories with a high risk of being harmful. For example, MYC1 and MYC2 have a high possibility of a variation of a base sequence being a driver variation. MYC1 has a higher risk of being harmful than MYC2, indicating that it is more likely to be a true driver variation with higher probability.

MYC3 is a category with a lower risk of being harmful than MYC1 and MYC2. For example, MYC3 is a category indicating that a variation of a base sequence has been evaluated assuming that it has a low possibility of being a driver variation (and therefore is not treated as a driver variation candidate). That is, MYC3 is a category indicating that the sequence variation is evaluated as a variation that is not harmful.

MYC4 is a category with a lower risk of being harmful than MYC3. MYC4 is a category indicating, for example, an evaluation that a possibility of a base sequence variation being a driver variation is almost 0, a known SNP, and a variation of a region where an error is likely to occur.

Fig. 5 is a block diagram illustrating an example of a detailed functional configuration of the first filter processing unit 23. In Fig. 5, the first filter processing unit 23 is provided with a basic filter 231, a time-series filter 232, a database filter 233, a function prediction filter 234, and a quality filter 235.

### <Basic filter>

in a case where a sequence variation to be analyzed can be determined to be benign, the basic filter 231 sets a category (for example, MYC4) indicating that the sequence variation is benign. In addition, in a case where it cannot be determined that the sequence variation to be analyzed is benign, the basic filter 231 determines that there is a risk of being harmful and sets a category (for example, MYC3) indicating that the sequence variation is not benign.

Here, the case where it can be determined as being benign corresponds to a case where a duplication portion between a base sequence of a known variation that causes canceration or the like and a base sequence corresponding to a sequence variation is a relatively short duplication portion, a case where a region where a variation represented by a sequence variation is located is an intron region, a case where a sequence variation is registered in a database in which variations without abnormality are accumulated, such as an SNP database, or a case where a sequence variation can be determined as being benign on the basis of a GDI (Gene Damage Index).

Here, a GDI is an index indicating how much damage is accumulated in a healthy person for each gene, and indicates a possibility that a gene is not considered to have a risk of being harmful due to a variation even if the gene is greatly damaged (even if there is diversity) depending on people.

The basic filter 231 receives, from the first setting reception unit 22, at least one setting of a threshold value of a length of a duplication portion between a base sequence of a known variation that causes canceration or the like and a base sequence that has varied corresponding to a sequence variation, information for specifying a database for determining whether the base sequence is SNP, and a parameter (which is compared with a value registered as a benign determination threshold value serving as a reference for determining whether or not the sequence variation is benign, a probability of SNP in a database, or the like) for each database. The basic filter 231 determines whether or not the sequence variation to be analyzed is benign on the basis of the received setting.

For example, in a case where the sequence variation is located at a site called segmental duplication, the basic filter 231 sets a category indicating that the sequence variation is a benign variation. Segmental duplication is gene duplication in a region where 10 to 300 kb of chromosomes are collected at adjacent sites where genes are replicated in the course of vertebrate evolution, or gene duplication on another genome that is completely separate. In a case where a sequence variation is located in the segmental duplication, it is considered that the sequence variation is a detection error generated at the time of mapping a sequence result to the reference, and there is a high possibility of being false positive. Therefore, in a case where the sequence variation is located in a segmental duplication region, a process of regarding the sequence variation as a benign variation is performed. Specifically, in a case where the sequence variation is located in the segmental duplication region and an index of the segmental duplication region exceeds a threshold value, there is a high possibility of an error, and thus a category indicating a benign variation is set. In addition, in a case where a region where a variation represented by a sequence variation is located is an intron region, the basic filter 231 sets a category indicating that the variation is a benign variation.

Furthermore, even if the above two conditions are not satisfied, the basic filter 231 may set a category indicating that the variation is a benign variation on the basis of the result of searching a designated SNP database. For example, in a case where the variation represented by the sequence variation is registered in the SNP database through the search, and a value registered as a probability of being the SNP exceeds a benign determination threshold value set in advance for the SNP database, the basic filter 231 sets a category indicating that the variation is a benign variation.

In addition, even in a case where the above conditions are not satisfied, the basic filter 231 refers to a GDI of a gene in which the sequence variation is present, and sets a category indicating that the variation is a benign variation when the GDI is more than a preset GDI threshold value.

As a result, the information processing apparatus 1 can screen in advance, for example, a gene that cannot be (or is sufficiently unlikely to be) a driver variation of cancer.

In addition, the basic filter 231 may receive, from the first setting reception unit 22, a setting as to which condition is used (alternatively, whether or not to pass the process by setting a category to MYC3 for all sequence variations without using all conditions and operating as the basic filter 231) from a plurality of preset conditions for determining that a variation is benign.

In this example, the basic filter 231 determines whether or not only a condition set to be used is satisfied.

### <Time-series filter>

In a case where the basic filter 231 passes the process (MYC3 is set), the time-series filter 232 refers to the information regarding a sequence variation included in the time-series information corresponding to a sequence variation to be analyzed, and determines whether or not the same variation has been present in time-series information extracted at different timings.

The time-series filter 232 uses the sequence variation to be analyzed and the corresponding sequence variation included in the time-series information, and, sets, in a case where the same variation is present, a category (for example, "1" is subtracted as a first predetermined amount from the current category) assuming that there is a variation to be a problem, and passes the process to the quality filter 235. The first predetermined amount is, for example, a minimum value subtracted or added in one calculation from a category related to a sequence variation. In this example, since the basic filter 231 has passed the process, the initial category is MYC3, and when the time-series filter 232 assumes that there is a variation to be a problem, "1" is subtracted from MYC3 as the first predetermined amount, and the category is set to MYC2.

On the other hand, the time-series filter 232 uses the sequence variation to be analyzed and the corresponding sequence variation included in the time-series information, sets the category without any change (here, since the initial category is MYC3, the category is set to MYC3 without any change) when the same variation is not present, and passes the process to the database filter 233.

Note that the time-series filter 232 may receive, from the first setting reception unit 22, a setting of threshold values regarding a depth, other sequence quality, a variant allele frequency, and the like. For example, in a case where the depth related to the corresponding sequence variation included in the time-series information does not exceed a threshold value (for example, "20") set here, the time-series filter 232 sets the category without any change without determining whether or not there is the same sequence variation (here, since the initial category is MYC3, the category is set to MYC3 without any change), and passes the process to the database filter 233.

Furthermore, in the example of the present embodiment, in a case where the time-series information is not included in the first base sequence information received by the first data reception unit 21, the time-series filter 232 may set the category without any change (here, since the initial category is MYC3, the category is set to MYC3 without any change) and pass the process to the database filter 233 without determining whether or not there is the same sequence variation.

In addition, in a case where a setting of not using the time-series filter 232 is input from the first setting reception unit 22, the time-series filter 232 sets the category without any change without determining whether or not there is the same sequence variation (here, since the initial category is MYC3, the category is set to MYC3 without any change), and passes the process to the database filter 233.

### <Database filter>

The database filter 233 checks whether or not a sequence variation to be analyzed is registered in a database (for example, a COSMIC cancer database or the like) in which information regarding a preset variation to be a problem is accumulated, by communicating with a server of the database. In a case where the sequence variation is registered in the database, a category (for example, "1" is subtracted as a first predetermined amount from the current category) is set on the assumption that there is a variation to be a problem (there is a risk of being harmful), and the process is passed to the quality filter 235. Here, an example of a series of processes in each filter will be described. In a case where the basic filter 231 passes the process assuming that there is a risk of being harmful for the sequence variation to be analyzed and the time-series filter 232 passes the process in a state in which the category is not changed, when the database filter 233 determines that there is a risk of being harmful, the database filter 233 subtracts "1" as the first predetermined amount from MYC3, sets the category to MYC2, and then passes the process to the quality filter 235.

In addition, in a case where the sequence variation to be analyzed is not registered in the database in which the information regarding the above variation to be a problem is accumulated, the database filter 233 sets the category without any change and passes the process to the function prediction filter 234. In this example, the category in this case is still MYC3.

Note that the database filter 233 receives, from the first setting reception unit 22, a setting as to what kind of database is to be used as a database that accumulates information regarding the above variation to be a problem.

In this setting, an instruction to use a plurality of databases may be given. In this case, in a case where the sequence variation to be analyzed is registered in any of the databases accumulating information regarding the above variation to be a problem, the database filter 233 sets the category assuming that there is the variation to be a problem.

### <Function prediction filter>

The function prediction filter 234 refers to a program (including a machine learning program) for evaluating or predicting a risk of a variation being harmful, and a database that discloses evaluation results or prediction values of the risk of being harmful. Then, in a case where the sequence variation to be analyzed is registered in a program or a database assuming that there is a risk of being harmful, a category (for example, "1" is subtracted as a first predetermined amount from the current category) is set assuming that the sequence variation has a variation having the risk of being harmful, and the process is passed to the quality filter 235.

Here, as a program for evaluating the risk of the variation being harmful, there are widely known programs such as SIFT, PolyPhen2, SnpEff, and VEP. In some of these programs and databases, a threshold value based on a score is provided for the presence or absence of a risk of being harmful, or evaluation is performed in multiple stages. For example, even in a case where the presence or absence of a risk of being harmful is in a determination stage in these programs and databases, the function prediction filter 234 sets a category (for example, "1" is subtracted as a first predetermined amount from the current category) assuming that there is a risk of being harmful, and passes the process to the quality filter 235.

In addition, the function prediction filter 234 may predict whether deletion or duplication of a promoter involved in important gene expression, deletion or insertion that leads to an abnormality in splicing of an important gene, deletion or insertion of noncoding RNA important for important gene expression control, or the like is caused by referring to the above-described programs or databases. In a case where the presence or absence of the risk of being harmful is in the determination stage in these programs, the function prediction filter 234 may set a category (for example, "1" as the first predetermined amount is subtracted from the current category) assuming that there is the risk of being harmful and pass the process to the quality filter 235.

Here, as an example of a series of processes in each filter, for a sequence variation to be analyzed, when the basic filter 231 passes the process assuming that there is a risk of being harmful, the time-series filter 232 passes the process in a state in which the category is not changed, and the database filter 233 also passes the process in a state in which the category is not changed, in a case where the function prediction filter 234 determines that there is a risk of being harmful, the function prediction filter 234 subtracts "1" as the first predetermined amount from MYC3 at that time, sets the category to MYC2, and then passes the process to the quality filter 235.

In addition, the function prediction filter 234 refers to the database that has evaluated the risk of the variation being harmful, and, sets, if the variation related to the sequence variation to be analyzed is not registered in the database assuming that there is the risk of being harmful (or is registered as a case where the variation is unknown even if it is registered, or is registered as a case where the variation is benign or estimated to be benign), the category without any change, and passes the process to the quality filter 235. In this example, the category in this case is still MYC3.

Note that it is assumed that the function prediction filter 234 also receives the setting as to what kind of database is used from the first setting reception unit 22.

### <Quality filter>

The quality filter 235 evaluates the quality of the sequence processing by using an index such as a depth when a sequence variation to be analyzed is sequenced, a quality score (for example, Phred Quality Score) for each base, a mapping quality score to a reference genome, a statistical value of a statistical test (Fisher's test or the like) in a variation call between a cancer cell and a normal cell, or the degree of bias to either side of a read sequence supporting a variation in a pair end read in which a base sequence is read from both sides. As an index of the quality, there is a widely known index such as the count number of sequence variation in addition to the depth, and the quality filter 235 evaluates the quality by combining these indexes (alternatively, a combination thereof is received from the first setting reception unit 22, and the quality is evaluated according to the combination of the received indexes). In the case of combining a plurality of indexes, the quality filter 235 determines that the quality is sufficient when a condition that the quality is sufficiently high is satisfied by all the indexes.

When it is determined that the quality of the sequence processing of the sequence variation to be analyzed is sufficient (sufficiently high) through this evaluation, the quality filter 235 sets a category (for example, "1" is subtracted as the first predetermined amount from the current category) assuming that the determination is appropriate, and outputs the category to the category determination unit 24. In addition, when it cannot be determined that the quality of the sequence processing of the sequence variation to be analyzed is sufficient (sufficiently high), the quality filter 235 sets the category without any change and outputs the category to the category determination unit 24.

At least one of the classification criteria provided in each filter can be changed or selected. Furthermore, it is also possible to execute the filtering unit 2 and the control unit 3 after changing or selecting at least one of the classification criteria. As a result, the information processing apparatus 1 can more accurately determine a risk of a sequence variation being harmful.

### (Category determination unit)

The category determination unit 24 determines a category value indicating the degree of risk of being harmful for each sequence variation according to the category (any one of MYC1 to MYC4) of each of one or more sequence variations output by the filter processing unit. The category determination unit 24 generates information (hereinafter, referred to as "analysis result information") in which the category values are respectively associated with the plurality of sequence variations, and provides the information to the analysis result output unit 25.

Note that, the category value representing the degree of the risk of being harmful may be a new value calculated on the basis of MYC1 to MYC4, but here, for convenience of description, MYC1 to MYC4 are adopted without any change.

### (Analysis result output unit)

The analysis result output unit 25 outputs the analysis result information by outputting the analysis result information from the display unit 14 (for example, a display) in Fig. 1 or transmitting the analysis result information from the communication unit 13 to another device (not illustrated).

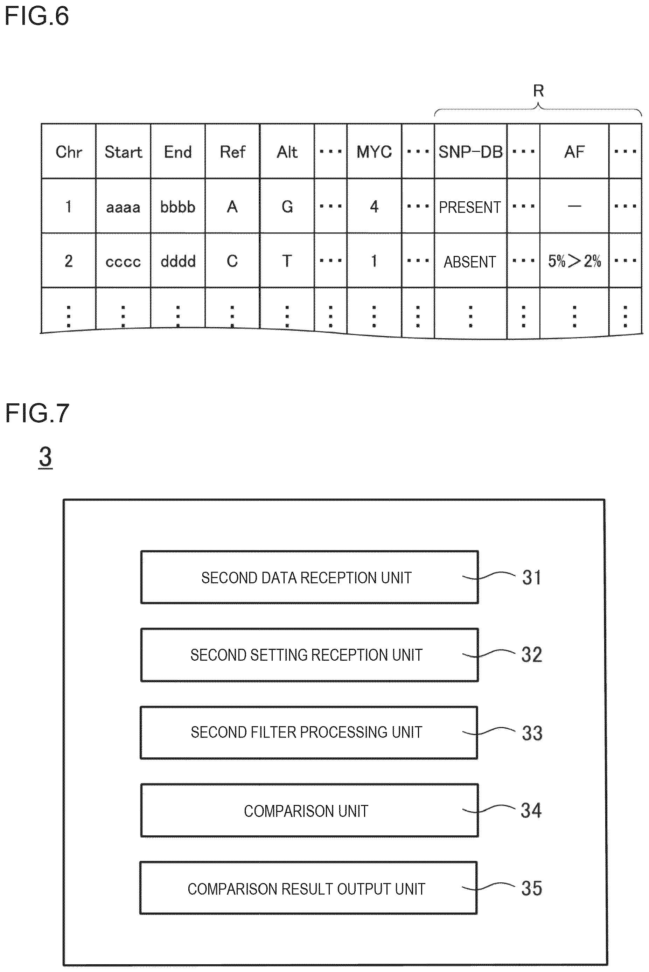

Fig. 6 illustrates a configuration example of analysis result information output from the information processing apparatus 1. As illustrated in Fig. 6, the analysis result information is information in which, for each sequence variation (for each row in the drawing), at least a number (Chr) of a chromosome in which a base sequence of the sequence variation is located, a start position (Start), an end position (End), the base sequence (Ref) that should originally be present, the sequence variation (Alt), and the category value (MYC) are associated with each other.

The analysis result information in the example in Fig. 6 is further associated with record information R related to determination for each sequence variation (for each row in the drawing).

The record information R related to determination is information indicating what kind of classification has been performed in a filter used for the analysis of a target sequence variation in the filter processing unit (parameter setting of each filter, determination content based on the classification criterion, and the like).

As described above, variations in the base sequence information received by the first data reception unit 21 are classified into four stages MYC1 to MYC4 indicating a risk of being harmful, and thus a user such as a medical specialist can efficiently find a variation having a high risk of being harmful, for example, a true driver variation, from among many (for example, tens of thousands to hundreds of millions) variations. For example, a user such as an expert can find a true driver variation by focusing on sequence variations classified as MYC1 and MYC2.

### <Control unit>

On the other hand, in order to improve the reliability of classification in the information processing apparatus 1, it is necessary to check whether the above-described classification process is appropriately performed. Therefore, the information processing apparatus 1 according to the present embodiment includes the control unit 3 that classifies a base sequence including a sequence variation of which a category to which the base sequence or the sequence variation is to belong is known into each of the categories on the basis of at least one of the above-described classification criteria, and compares a result of the classification with the category to which the base sequence or the sequence variation is to belong. In a case where a comparison result indicates matching, it can be checked that the classification process of the information processing apparatus 1 is appropriately performed. On the other hand, in a case where the comparison result indicates mismatching, it can be checked that there is a possibility that the classification process of the information processing apparatus 1 is not appropriately performed.

The control unit 3 according to the present embodiment classifies a base sequence including a sequence variation of which a category to which the sequence variation is to belong is known into each of categories according to the degree of the risk of being harmful on the basis of at least one of classification criteria, and compares the classification result with the category to which the sequence variation is to belong.

Fig. 7 is a block diagram illustrating an example of a functional configuration for executing various processes related to the control unit 3 in the information processing apparatus 1. As illustrated in Fig. 7, the control unit 3 functions as a second data reception unit 31, a second setting reception unit 32, a second filter processing unit 33, a comparison unit 34, and a comparison result output unit 35.

### (Second data reception unit)

The second data reception unit 31 receives base sequence information (hereinafter, also referred to as second base sequence information) including information indicating a base sequence including one or more sequence variations of which categories to which the sequence variations are to belong are known. Here, a base sequence including a sequence variation of which a category to which the sequence variation is to belong is known includes a sequence variation of which a category to which the sequence variation is to belong is known and a base sequence of which a category to which the base sequence is to belong is known but does not have a variation. The category to which a base sequence is to belong is any of MYC1, MYC2, MYC3, and MYC4, which are categories according to the degree of the risk of being harmful described above.

A configuration example of the second base sequence information received by the second data reception unit 31 is equivalent to the configuration example of the first base sequence information received by the first data reception unit 21 illustrated in Fig. 4, and thus description thereof will be omitted. However, the base sequence information received by the second data reception unit 31 includes information regarding a category to which each sequence variation is to belong.

In one embodiment, a base sequence including a sequence variation of which a category to which the sequence variation is to belong is known may have two or more types of different categories to which the sequence variation is to belong. In the control unit 3, filter processing is performed on two or more types of base sequences having different categories to which the base sequences are to belong, so that a comparison result from the comparison unit 34 that will be described later becomes more detailed, and the accuracy of the filter processing can be ascertained in more detail.

In addition, in one embodiment, two or more types of base sequences to be classified by the control unit 3 and having different categories to which the base sequences are to belong may include a sequence variation that causes a specific disease (there is a risk of being harmful) and a base sequence that does not cause a specific disease (there is no risk of being harmful). Here, the base sequence that does not cause a specific disease includes a sequence variation having no risk of being harmful and a base sequence having no variation. For example, a base sequence including a sequence variation that causes a specific cancer and a base sequence not including a sequence variation that causes the specific cancer are processed by the control unit 3. Consequently, it is possible to determine whether the determination function of the second filter processing unit 33 is working normally for both cases where there is a risk of being harmful and where there is no risk of being harmful.

In addition, the second base sequence information may include files such as a VCF (Variant Call Format) format, a FASTQ format, a SAM (Sequence Alignment Map) format, and a BAM (Binary Alignment Map) format, which are output from a next-generation sequencer or the like. The VCF format is a file format used when base variation data is stored, in which, when sequencing data is mapped to a reference sequence, information such as a base on the reference sequence and a base on the sequencing data mapped thereto is written. A file in FASTQ format includes a base sequence and the quality of base calls per base. A file in the SAM format is a file indicating a result of mapping a read sequence of FASTQ to a sequence serving as a reference, and the BAM format is a format obtained by compressing the SAM format so that a computer can easily process the BAM format.

These files may represent base sequences including any sequence variations, and it is possible to more accurately classify any sequence variations by providing such files to the control unit 3. More specifically, for example, in a case where any sequence variation is a hot spot where variations concentrate in a gene, it is possible to more accurately classify variations in the hot spot by providing the files including information regarding the hot spot to the control unit 3. As a result, it is possible to more reliably classify the variations in the hot spot in the filtering unit 2.

Further, in one embodiment, in a case where a target sequence variation having a risk of being harmful on a base sequence of a subject is a driver variation of a specific disease, the base sequence including two or more types of sequence variations whose categories to which the sequence variations are to belong are different from each other may include a sequence variation that becomes a driver variation of the specific disease and a base sequence that does not become a driver variation of the specific disease. For example, in a case where a target sequence variation on a base sequence of a specimen obtained from a patient is a driver variation of a certain leukemia, a sequence variation that is the driver variation of the leukemia and a base sequence not including the driver variation of the leukemia are processed by the control unit 3. Consequently, it is possible to ascertain whether the information processing apparatus 1 accurately classifies the driver variation of the specific disease.

### (Second setting reception unit)

The second setting reception unit 32 receives a setting for analyzing the second base sequence information received by the second data reception unit 31. This setting includes, for example, a setting as to a classification criterion based on which a filter is used in the second filter processing unit 33 that will be described later.

Similarly to the filtering unit 2, the control unit 3 performs evaluation regarding a risk of being harmful (for example, a possibility of a driver variation) based on various types of information in which the base sequence information received by the second data reception unit 31 affects the interpretation of an analysis result of a variation through an operation of the second filter processing unit 33. This evaluation result is also classified into any of the categories MYC1 to MYC4, similarly to the evaluation result in the filtering unit 2. An evaluation (classification) in the second filter processing unit 33 and information that affects interpretation are similar to those of the filtering unit 2, and thus description thereof will be omitted.

### (Second filter processing unit)

The second filter processing unit 33 classifies a base sequence including a sequence variation of which a category to which the sequence variation is to belong is known, included in the base sequence information received by the second data reception unit 31, into any of MYC1, MYC2, MYC3, and MYC4 that are categories according to the degree of risk of being harmful, on the basis of at least one classification criterion. MYC1, MYC2, MYC3, and MYC4 are as described in the section of the first filter processing unit 23. Although the second filter processing unit 33 is described separately from the first filter processing unit 23 for convenience of description in the present specification, each classification criterion and each filter used in the second filter processing unit 33 may be common to the first filter processing unit 23, and the second filter processing unit 33 and the first filter processing unit 23 may be common filter processing units.

### (Comparison unit)

The comparison unit 34 compares, for each variation of the base sequence information received by the second data reception unit 31, the category (any of MYC1 to MYC4) output by the second filter processing unit 33 with the known category (any of MYC1 to MYC4) corresponding to the degree of risk of being harmful. In addition, the comparison unit 34 provides the comparison result output unit 35 with a comparison result of each variation.

Note that a value representing this comparison result may be a new value calculated on the basis of MYC1 to MYC4, but here, for convenience of description, MYC1 to MYC4 are assumed to be adopted without any change.

### (Comparison result output unit)

The comparison result output unit 35 outputs information regarding the comparison result from the comparison unit 34 by outputting the information from the display unit 14 (for example, a display) in Fig. 1 or transmitting the information from the communication unit 13 to another device (not illustrated).

### <Adjustment unit>

The information processing apparatus 1 according to an embodiment may include the adjustment unit 4 that adjusts the classification criteria in the filtering unit 2 and/or the control unit 3 and/or the classification result in the filtering unit 2 on the basis of the comparison result from the control unit 3. By including the adjustment unit 4, the information processing apparatus 1 can perform calibration of criteria and the like in filter processing, and can thus more accurately classify the degree of risk of a variation of a base sequence of a subject being harmful.

For example, as a result of comparison in the comparison unit 34 of the control unit 3, in a case where a category output by the filter processing unit and the known category corresponding to the degree of risk of being harmful are different for a certain sequence variation, the degree of risk of the sequence variation being harmful is not accurately classified in the filter processing unit. In such a case, the adjustment unit 4 calibrates the classification criteria and the like of each filter of the filter processing unit on the basis of the comparison result so that the category output by the filter processing unit matches the known category.

In addition, as a result of the comparison in the comparison unit 34 of the control unit 3, in a case where the category output by the filter processing unit and the known category corresponding to the degree of risk of being harmful do not match for a certain sequence variation, the adjustment unit 4 may perform the classification process using the filtering unit 2 again after adjustment in the adjustment unit 4 is ended without adopting the classification result from the filtering unit 2. In addition, the adjustment unit 4 may have a function of displaying the content of a generated problem in an error message on the basis of a result of comparison in the comparison unit 34 of the control unit 3. For example, it is possible to display at which stage of the filter processing the problem has occurred.

In addition, a sequence variation to be classified by the control unit 3 may be obtained by sequencing a standard composition of nucleic acids including a sequence variation of which a category to which the sequence variation is to belong is known. That is, the standard composition of nucleic acids including a sequence variation of which a category to which the sequence variation is to belong is known may be sequenced by a sequence using a sequencing apparatus such as a next generation sequencer, and information regarding a result of the sequencing may be subjected to processing of the control unit 3. The control unit 3 classifies information regarding the result of sequencing the standard composition, and compares the result of the classification with a known category to which the standard composition should originally belong, so that it is possible to check whether or not sequencing conditions (for example, sequencing in a sequencing apparatus, a preprocessing step of sequencing, and the like) are correct.

In this case, a condition for sequencing the standard composition and a condition for sequencing nucleic acids included in a subject may be the same. For example, a condition for sequencing the standard composition with a next generation sequencer or the like and a condition for sequencing nucleic acids included in a subject derived from a patient or the like may be the same. As described above, it is possible to check whether or not the sequencing conditions were correct by providing the control unit 3 with the result of sequencing the standard composition to which the category to which the standard composition is to belong is known. Therefore, by setting the conditions for sequencing the nucleic acids included in the subject and the standard composition to be the same, it is also possible to check whether or not the conditions for sequencing the nucleic acids included in the subject are correct.

Hereinafter, adjustment of each filter in the adjustment unit 4 will be described by way of specific examples, but the adjustment in the adjustment unit 4 is not limited thereto.

### <<Example of adjustment of basic filter>>

In the basic filter 231, the adjustment unit 4 can adjust a threshold value of a length of a duplication portion between a base sequence having a known variation that causes canceration or the like and a base sequence corresponding to the sequence variation. For example, in a case where the sequence variation is located in the segmental duplication region and an index of the segmental duplication region exceeds a threshold value, there is a high possibility of an error. Therefore, the basic filter 231 sets a category indicating that the sequence variation is a benign variation, but can adjust this threshold value. As a result, the basic filter 231 can adjust the classification criterion for setting a category.

In addition, the adjustment unit 4 can change the SNP database used in the basic filter 231. Further, the adjustment unit 4 can also set a plurality of SNP databases to be used in the basic filter 231. In addition, in the basic filter 231, in a case where a variation represented by a sequence variation is registered in the SNP database and a value registered in the database as a probability of being SNP exceeds a benign determination threshold value in the basic filter 231, a category representing a benign variation is set, but the adjustment unit 4 can change the benign determination threshold value in the basic filter 231. Through this adjustment, the basic filter 231 can also adjust the classification criterion for setting a category indicating a benign variation.

The basic filter 231 refers to a GDI of a gene in which the sequence variation is present, and sets a category indicating a benign variation in a case where the GDI is more than a predetermined GDI threshold value, but the adjustment unit 4 can also adjust the GDI threshold value. Through this adjustment, the adjustment unit 4 can also adjust the classification criterion for the basic filter 231 setting a category.

In addition, the adjustment unit 4 can change a condition to be used (alternatively, whether or not to pass the process by setting a category to MYC3 for all sequence variations without using all conditions and operating as the basic filter 231) from a plurality of conditions for determining that a variation is benign, set in advance in the first setting reception unit 22 or the like.

### <<Example of adjustment of time-series filter>>

The time-series filter 232 uses a sequence variation to be analyzed and a corresponding sequence variation included in time-series information, and sets, in a case where the same variation is present, a category assuming that there is a variation to be a problem. Here, for example, in a case where a plurality of pieces of time-series information are included, the adjustment unit 4 can perform adjustment such that the control unit 3 uses time-series information different from the time-series information used for the time-series filter 232.

In addition, in a case where the time-series filter 232 has received in advance settings of threshold values regarding a depth, other sequence quality, variant allele frequency, and the like, the adjustment unit 4 can adjust these settings. For example, it is also possible to adjust a category classified by the time-series filter 232 by changing a threshold value of a depth related to a corresponding sequence variation included in time-series information.

### <<Example of database filter adjustment>>

The database filter 233 checks whether or not a sequence variation to be analyzed is registered in a database storing information regarding variations by transmitting information regarding the sequence variation to a server of the database. In a case where the sequence variation is registered, a category is set assuming that there is a variation to be a problem. The adjustment unit 4 can change a database to be used in the database filter 233. As a result, the adjustment unit 4 can also adjust a category set by the database filter 233.

### <<Example of adjustment of function prediction filter>>

The function prediction filter 234 refers to a program or database that has evaluated a risk of a variation being harmful, and, sets, in a case where a sequence variation to be analyzed is registered in the database assuming that there is a risk of being harmful, a category assuming that a variation has a risk of being harmful. The adjustment unit 4 can perform setting such that a program or database different from the program or database referred to can be referred to, and can thus adjust a category set by the function prediction filter 234.

### <<Example of adjustment of quality filter>>

The quality filter 235 evaluates the quality of sequence processing of a sequence variation to be analyzed by using an index related to quality such as a depth when the sequence variation to be analyzed is sequenced, a quality score (for example, Phred Quality Score) for each base, a mapping quality score to a reference genome, a statistical test (such as Fisher's test) in a variation call between a cancer cell and a normal cell, and a statistical value of a bias degree of support read of a variation in pair end read. The adjustment unit 4 can adjust a category set by the quality filter 235 by changing the evaluation criteria of these indexes indicating the quality of the sequence.

The method of adjusting the classification criteria for each filter in the adjustment unit 4 has been described above. Note that the information processing apparatus 1 according to an embodiment may include a re-execution unit that re-executes processing of the filtering unit 2 and the control unit 3 after the adjustment unit 4 changes or selects at least one of these classification criteria. As a result, classification using the calibrated classification criterion or filter can be performed, so that the accuracy of classification of the information processing apparatus 1 is improved.

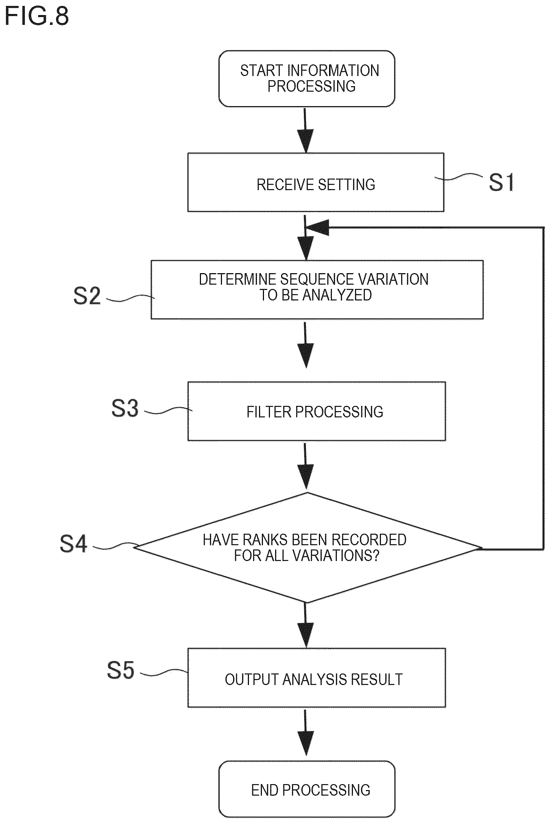

Next, processing of the information processing apparatus 1 will be described with reference to Fig. 8 and subsequent drawings.

Fig. 8 is a flowchart illustrating an example of a series of flows in the filtering unit 2 of the information processing apparatus 1 having the functional configuration in Fig. 3.

In step S1, the first setting reception unit 22 receives a setting for analyzing base sequence information. Here, the first filter processing unit 23 also receives a setting as to what kind of classification criterion a filter is to be used based on.

In step S2, the first data reception unit 21 determines a predetermined sequence variation in base sequence information extracted from genetic information of a subject to be analyzed through sequence alignment as a processing target.

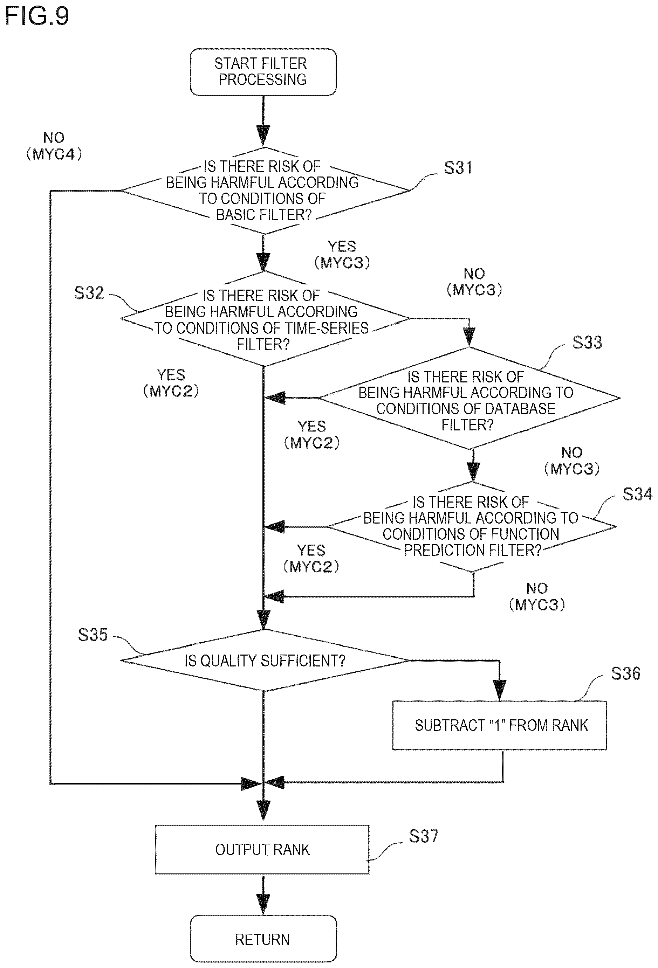

In step S3, the first filter processing unit 23 performs filter processing on the sequence variation that is the processing target, thereby outputting a category of the processing target. Details of the filter processing in the first filter processing unit 23 will be separately described with reference to Fig. 9.

Subsequently, in step S4, the information processing apparatus 1 determines whether or not categories have been recorded for all sequence variations.

in a case where there is a sequence variation of which a category is not recorded, "NO" is determined in step S4, the process returns to step S2, and the subsequent processes are repeatedly performed. As described above, as a result of repeatedly performing the loop processing of "NO" in steps S2 to S4, in a case where all the categories of sequence variations have been recorded, "YES" is determined in step S4, and the process proceeds to step S5.

In step S5, the analysis result output unit 25 generates analysis result information and outputs the analysis result information from the display unit 14 (for example, a display) in Fig. 1 or outputs the analysis result information by transmitting the analysis result information from the communication unit 13 to another device (not illustrated). As a result, the analysis process is ended.

Details of the filter processing in step S3 will be described below with reference to a flowchart of Fig. 9.

In step S31, the basic filter 231 determines whether or not there is a risk of being harmful for the sequence variation to be processed according to conditions of the basic filter 231.

In a case where the sequence variation to be processed has no risk of being harmful according to the conditions of the basic filter 231, "NO" is determined in step S31, a category is set to MYC4, and the process proceeds to step S37 or step 35.

In a case of proceeding to step S37, the first filter processing unit 23 outputs the category as the first filter processing unit 23. As a result, the filter processing in step S3 in Fig. 9 is ended, and the process proceeds to step S4. Note that a process in the case of proceeding to step S35 will be described later.

In a case where the sequence variation to be processed has a risk of being harmful according to the condition of the basic filter 231, "YES" is determined in step S31, the category is set to MYC3, and the process proceeds to step S32.

In step S32, the time-series filter 232 determines whether or not there is a risk of being harmful for the sequence variation to be processed according to the conditions of the time-series filter 232. In a case where the sequence variation to be processed has a risk of being harmful according to the conditions of the time-series filter 232, "YES" is determined in step S32, the category is set to MYC2, and the process proceeds to step S35. Note that the processes in and after step S35 will be described later.

In a case where the sequence variation to be processed has no risk of being harmful according to the conditions of the time-series filter 232, "NO" is determined in step S32, the category is set to MYC3, and the process proceeds to step S33.

In step S33, the database filter 233 determines whether or not there is a risk of being harmful for the sequence variation to be processed according to the conditions of the database filter 233.

When the sequence variation to be processed has a risk of being harmful according to the condition of the database filter 233, "YES" is determined in step S33, the category is set to MYC2, and the process proceeds to step S35. Note that the processes in and after step S35 will be described later.

In a case where the sequence variation to be processed has no risk of being harmful according to the conditions of the time-series filter 232, "NO" is determined in step S33, the category is set to MYC3, and the process proceeds to step S34.

In step S34, the function prediction filter 234 determines whether there is a risk of being harmful for the sequence variation to be processed according to the conditions of the function prediction filter 234.

In a case where the sequence variation to be processed has a risk of being harmful according to the conditions of the function prediction filter 234, "YES" is determined in step S34, the category is set to MYC2, and the process proceeds to step S35.

In a case where the sequence variation to be processed has no risk of being harmful according to the conditions of the function prediction filter 234, "NO" is determined in step S34, the category is set to MYC3, and the process proceeds to step S35.

In step S35, the quality filter 235 determines whether or not the quality is sufficient.

In a case where the quality of the results of the processes in steps S31 to S34 (filtering results in the basic filter 231, the time-series filter 232, the database filter 233, and the function prediction filter 234) is sufficient, "YES" is determined in step S35, and the process proceeds to step S36. In step S36, since it is determined that the quality is sufficient, the quality filter 235 subtracts "1" that is the first predetermined amount from the category.

In a case where the quality of the results of the processes in steps S31 to S34 (filtering results in the basic filter 231, the time-series filter 232, the database filter 233, and the function prediction filter 234) is not sufficient, "NO" is determined in step S35, and the process proceeds to step S37.

In step S37, the first filter processing unit 23 outputs the category. As a result, the filter processing in step S3 in Fig. 9 is ended, and the process proceeds to step S4.

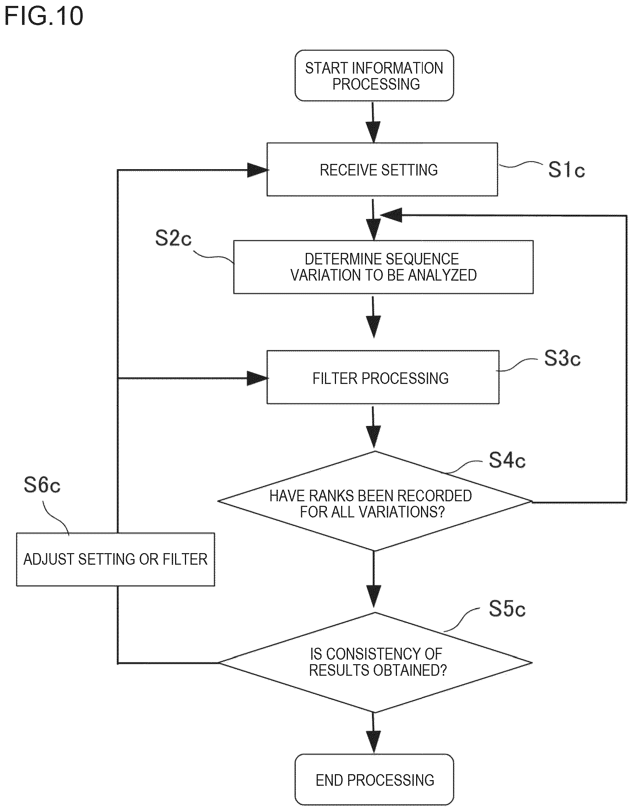

Fig. 10 is a flowchart illustrating an example of a series of flows in the control unit 3 and the adjustment unit 4 of the information processing apparatus 1 having the functional configuration in Fig. 7.

In step S1c, the second setting reception unit 32 receives a setting for analyzing the second base sequence information regarding the base sequence including the sequence variation of which the category to which the sequence variation is to belong is known. Here, the second filter processing unit 33 also receives a setting as to what kind of classification criterion a filter is to be used based on.

In step S2c, the second data reception unit 31 determines a base sequence to be analyzed. In a case where there are a plurality of base sequences, a base sequence to be analyzed is selected and determined from among a plurality of variations. Fig. 10 illustrates a case where the base sequence to be analyzed by the control unit 3 is a sequence variation of which a category to which the sequence variation is to belong is known. However, in the control unit 3, a base sequence of which a category to which the base sequence is to belong is known and that has no variation may be analyzed.

In step S3c, the second filter processing unit 33 performs filter processing on the sequence variation that is the processing target, thereby outputting a category of the processing target. The filter processing in the second filter processing unit 33 is similar to the filter processing in the first filter processing unit 23 described with reference to Fig. 9, and thus description thereof will be omitted.

In a case where the second base sequence information includes a plurality of sequence variations, in step S4c, the information processing apparatus 1 determines whether or not categories have been recorded for all the sequence variations. In a case where there is a sequence variation for which no category is recorded, "NO" is determined in step S4c, the process returns to step S2c, and the subsequent processes are repeatedly performed.

As described above, as a result of repeatedly performing the loop processing of "NO" in steps S2c to S4c, in a case where all the categories of sequence variations have been recorded, "YES" is determined in step S4c, and the process proceeds to step S5c.

Subsequently, in step S5c, for the sequence variation in the second base sequence information received by the second data reception unit 31, the category (any of MYC1 to MYC4) output by the second filter processing unit 33 is compared with the known category to which the sequence variation is to belong (any of MYC1 to MYC4). As a result of the comparison, in a case where consistency is obtained between the category output by the filter processing unit and the known category to which the sequence variation is to belong (for example, in a case where the categories coincide with each other), a result indicating that the consistency is obtained is output, and the processing of the control unit 3 is ended.

On the other hand, as a result of the comparison, in a case where consistency is not obtained between the category output by the filter processing unit and the known category to which the sequence variation is to belong for the sequence variation in the second base sequence information (for example, when the categories do not match), the adjustment unit 4 adjusts the classification criterion or the result of classification into each of the categories in step S6c. Details of an adjustment method in the adjustment unit 4 will be described in the section of the adjustment unit 4.

After the adjustment, the processes in steps S2c to S5c are performed again on the sequence variation included in the second base sequence information, and in a case where consistency is obtained in the comparison result between the category output by the filter processing unit and the known category corresponding to the degree of risk of being harmful, the processing of the control unit 3 is ended. Note that, in a case where the consistency in the comparison result is not obtained, the processes in steps S2c to S6c described above may be repeatedly performed, and the processing of the control unit 3 may be ended at the time at which the consistency is obtained.

Note that the processing of the filtering unit 2 may be executed after the processing of the control unit 3 is ended.

Although one embodiment of the present invention has been described above, the present invention is not limited to the above-described embodiment (also referred to as a first embodiment), and modifications, improvements, and the like within the scope of achieving the object of the present invention are considered to be included in the present invention.

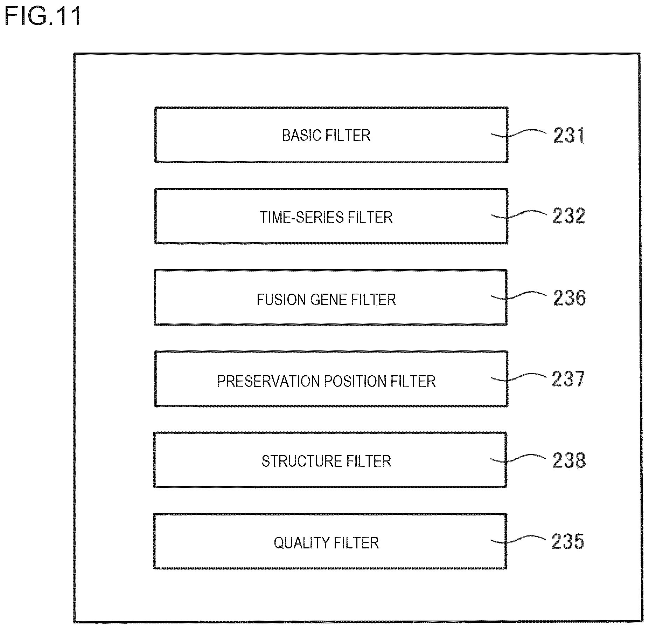

For example, the filter processing unit is not particularly limited to the examples of the first filter processing unit 23 and the second filter processing unit 33 illustrated in Fig. 5, and various forms having different filter configurations can be taken. Hereinafter, an information processing apparatus 1 employing a third filter processing unit 43 having a configuration illustrated in a block diagram in Fig. 11 will be described as a second embodiment of the information processing apparatus 1 according to the present invention. Note that the second embodiment of the information processing apparatus 1 has the same configuration as that of the first embodiment described above except for a configuration described below (for example, the third filter processing unit 43 and the adjustment unit 4 that adjusts the third filter processing unit 43), and thus the description of the same configuration as that of the first embodiment will be omitted.

The third filter processing unit 43 in the example in Fig. 11 is useful in the following sequence variation analysis.

First, as a premise, it is known that two genes of a specific combination are fused due to chromosomal translocation, inversion, or the like, thereby causing proliferation of cancer cells. For example, it is known that a BCR-ABL fusion gene in which a BCR gene and an ABL gene are fused due to chromosomal translocation proliferates leukemia cells.

The third filter processing unit 43 includes a basic filter 231, a time-series filter 232, a fusion gene filter 236, a preservation position filter 237, a structure filter 238, and a quality filter 235.

In addition, base sequences in which a plurality of combinations of candidate genes known to cause a driver variation are encoded in a fusion gene in which two candidate genes of a specific combination are fused are each stored in one region of the storage unit 12 for each fusion gene. For example, a base sequence in which a BCR gene and an ABL gene are encoded is stored in one region of the storage unit 12.

That is, the information processing apparatus 1 can acquire the following information and use the information for information processing.

The information processing apparatus 1 acquires, for each first fusion gene, base sequences of two candidate genes that are driver variation candidates in a fusion gene (hereinafter, a first fusion gene) in which candidate genes of a specific combination are fused. In the example in which the third filter processing unit 43 in Fig. 11 is adopted, the information processing apparatus 1 acquires a base sequence of each of the two candidate genes included in the plurality of first fusion genes stored in the storage unit 12 from the storage unit 12 for each first fusion gene.

In addition, an external server (not illustrated) may store a base sequence in which a plurality of candidate genes of the first fusion gene are encoded. The information processing apparatus 1 may acquire, for each first fusion gene, a base sequence in which two candidate genes of the first fusion gene are encoded from the external server via the communication unit 13.

A fusion gene in which a specific candidate gene is fused with other genes may cause proliferation of cancer cells. For example, it is known that a fusion gene in which the ALK gene is fused with other genes causes proliferation of cancer cells. The base sequences of a plurality of candidate genes that are driver variation candidates in a fusion gene (hereinafter, also referred to as a second fusion gene) fused with another gene are stored in the storage unit 12.

The information processing apparatus 1 acquires a base sequence of a candidate gene that is a driver variation candidate in the second fusion gene fused with another gene. For example, the information processing apparatus 1 acquires base sequences of candidate genes of a plurality of second fusion genes from the storage unit 12. The information processing apparatus 1 may acquire the base sequences of the plurality of candidate genes of the second fusion gene from an external server via the communication unit 13.

The information processing apparatus 1 acquires preserved sequence position information indicating a position of a preserved sequence that is a base sequence preserved between genomes of different species. For example, the information processing apparatus 1 acquires the preserved sequence position information from the storage unit 12. The information processing apparatus 1 may acquire the preserved sequence position information from an external server via the communication unit 13.

### <Basic filter>

The basic filter 231 is similar to one in the filter processing unit illustrated in Fig. 5 except that the processing specific to single nucleotide polymorphism is not executed. In a case where a sequence variation to be analyzed can be determined to be benign, the basic filter 231 sets a category (for example, MYC4) indicating that the sequence variation is benign, and outputs the result to a filter set as the next filter. If the sequence variation to be analyzed cannot be determined to be benign, the basic filter 231 sets a category (for example, MYC3) indicating that the sequence variation is not benign, and passes the process to a filter set as the next filter.

The basic filter 231 receives, from the first setting reception unit 22, information specifying a threshold value of a length of a duplication portion between a base sequence of a known variation that causes canceration and the like and a base sequence that has varied corresponding to the sequence variation, and a setting of a parameter for each database (which is compared with a value registered as a benign determination threshold value or the like serving as a reference for determining whether or not a sequence variation is benign), and determines whether or not a sequence variation to be analyzed is benign on the basis of the setting.

Specifically, in a case where the duplication portion between the base sequence of the known variation causing canceration or the like and the base sequence that has varied corresponding to the sequence variation is a duplication portion shorter than a preset length threshold value, the basic filter 231 sets a category indicating that the sequence variation is a benign variation. Even if not, the basic filter 231 sets a category indicating that the sequence variation is a benign variation in a case where a region where the variation represented by the sequence variation is located is an intron region.

Furthermore, even if the above two conditions are not satisfied, the basic filter 231 searches a designated database, and sets a category indicating that the sequence variation is a benign variation in a case where a variation represented by the sequence variation is registered in the database through the search, and a value registered as a probability of being the variation exceeds a benign determination threshold value set in advance for the database.

### <Time-series filter>

The time-series filter 232 is similar to the example of the filter processing unit in Fig. 5 except that a value to be subtracted from a category corresponding to a sequence variation to be analyzed is different from the example of the filter processing unit in Fig. 5, and an output destination of the category after calculation in the time-series filter 232 is different from the example of the filter processing unit in Fig. 5. The time-series filter 232 refers to information regarding a sequence variation included in the time-series information corresponding to a sequence variation to be analyzed, and determines whether or not there is the same variation in time-series information extracted at different timings.

The time-series filter 232 uses the sequence variation to be analyzed and the corresponding sequence variation included in the time-series information, determines a category (for example, "2" is subtracted from the category as a second predetermined amount) corresponding to the sequence variation to be analyzed assuming that there is a risk of being harmful in a case where the same variation is present, and passes the process to the structure filter 238. In this example, since the basic filter 231 passes the process, the initial category is MYC3, and here, when the time-series filter 232 determines that there is a risk of being harmful, "2" is subtracted as the second predetermined amount from MYC3, and the category is set to MYC1. The second predetermined amount is a value larger than the first predetermined amount.

On the other hand, the time-series filter 232 uses the sequence variation to be analyzed and the corresponding sequence variation included in the time-series information, sets the category without any change (here, since the initial category is MYC3, the category is set to MYC3 without any change) when the same variation is not present, and passes the process to the database filter 233.

Note that the time-series filter 232 may receive, from the first setting reception unit 22, a setting of threshold values regarding a depth, other sequence quality, a variant allele frequency, and the like. For example, in a case where the depth related to the corresponding sequence variation included in the time-series information does not exceed a threshold value (for example, "20") set here, the time-series filter 232 sets the category without any change without determining whether or not there is the same sequence variation (here, since the initial category is MYC3, the category is set to MYC3 without any change), and passes the process to the database filter 233.

Further, similarly to the example of the filter processing unit in Fig. 5, in a case where the first data reception unit 21 has not received the time-series information, the time-series filter 232 may set (here, since the initial category is MYC3, the category is set to MYC3 without any change) the category without any change and pass the process to the database filter 233 without determining whether or not there is the same sequence variation.

In addition, in a case where a setting of not using the time-series filter 232 is input from the first setting reception unit 22, the time-series filter 232 sets (here, since the initial category is MYC3, the category is set to MYC3 without any change) the category without any change without determining whether or not there is the same sequence variation, and passes the process to the fusion gene filter 236.

### <Fusion gene filter>

Hereinafter, a base sequence corresponding to any sequence variation included in base sequence information will also be referred to as a variant base sequence. The fusion gene filter 236 determines whether or not a fusion gene in which two genes respectively similar to the two candidate genes of the first fusion gene acquired by the information processing apparatus 1 are fused is included in the variant base sequence. More specifically, for the plurality of first fusion genes acquired by the information processing apparatus 1, the fusion gene filter 236 determines, for each of the first fusion genes, whether or not the similarity between the two base sequences in which the two candidate genes of the first fusion gene are encoded and at least a part of the base sequences included in the variant base sequence is equal to or more than a threshold value for each of the two base sequences. The similarity is represented by, for example, a ratio at which alignments of two base sequences match each other. In a case where the ratio at which the alignments of the two base sequences match is a threshold value or more, it is determined that the two base sequences are similar to each other.

As an example, the fusion gene filter 236 obtains the similarity between the base sequence in which the BCR gene is encoded in the BCR-ABL first fusion gene obtained by fusing the BCR gene and the ABL gene acquired by the information processing apparatus 1 and the corresponding base sequence in the variant base sequence. Next, the fusion gene filter 236 obtains the similarity between the base sequence in which the ABL gene is encoded in the BCR-ABL first fusion gene and the corresponding base sequence in the variant base sequence.

The fusion gene filter 236 determines whether or not both of the obtained two similarities are equal to or more than a threshold value. The threshold value is, for example, a value in which it is assumed that the activity of the protein in which the first fusion gene is encoded is similar to the activity of the protein indicated by the variant base sequence.

The fusion gene filter 236 determines that a fusion gene in which two genes respectively similar to the two candidate genes of the first fusion gene are fused is included in the variant base sequence in a case where the obtained two similarities are both equal to or more than the threshold value.

On the other hand, in a case where at least one of the obtained two similarities is less than the threshold value, the fusion gene filter 236 repeats the same determination for another first fusion gene acquired by the information processing apparatus 1. In a case where at least one of the obtained two similarities is less than the threshold value for all the first fusion genes acquired by the information processing apparatus 1, the fusion gene filter 236 determines that a fusion gene in which two genes respectively similar to two candidate genes of the first fusion gene are fused is not included in the variant base sequence for any first fusion gene.

In addition, in a case where each of the similarities between the base sequences of the two candidate genes of the first fusion gene acquired by the information processing apparatus 1 and the base sequences of the two genes of the fusion gene included in the variant base sequence is 65% or more and 100% or less, the fusion gene filter 236 may determine that the fusion gene in which the two genes respectively similar to the two candidate genes of the first fusion gene are fused is included in the variant base sequence. Preferably, when the similarity between the base sequences of the two candidate genes of the first fusion gene and the base sequences of the two genes of the fusion gene included in the variant base sequence is 80% or more and 100% or less, the fusion gene filter 236 may determine that a fusion gene in which two genes respectively similar to the two candidate genes of the first fusion gene are fused is included in the variant base sequence.

In addition, the fusion gene filter 236 may transmit a variant base sequence corresponding to the sequence variation to be analyzed to an external server that stores a combination of candidate genes of a plurality of first fusion genes. The fusion gene filter 236 checks whether or not a fusion gene of two genes respectively similar to two candidate genes of the first fusion gene registered in a database of the external server is included in the variant base sequence. When receiving, from the external server, a notification indicating that the fusion gene of the two genes respectively similar to the two candidate genes of any one of the first fusion genes among the plurality of first fusion genes registered in the database of the external server is included in the variant base sequence, the fusion gene filter 236 may determine that the fusion gene in which the two genes respectively similar to the two candidate genes of the first fusion gene are fused is included in the variant base sequence.

The fusion gene filter 236 determines whether or not a fusion gene in which a gene having a base sequence similar to the base sequence of the candidate gene of the second fusion gene acquired by the information processing apparatus 1 and another gene are fused is included in the variant base sequence. More specifically, for the plurality of second fusion genes acquired by the information processing apparatus 1, the fusion gene filter 236 obtains a similarity between the base sequence of the candidate gene of the second fusion gene and the base sequence of one gene of the fusion genes included in the variant base sequence for each second fusion gene. The fusion gene filter 236 determines whether or not the obtained similarity is equal to or more than a threshold value. The threshold value is a value in which it is assumed that the activity of the protein in which the second fusion gene is encoded is similar to the activity of the protein indicated by the variant base sequence.

In a case where the obtained similarity is equal to or more than the threshold value, the fusion gene filter 236 determines that the fusion gene of the gene similar to the candidate gene of the second fusion gene acquired by the information processing apparatus 1 is included in the variant base sequence. In a case where the obtained similarity is less than the threshold value, the fusion gene filter 236 repeats the same determination for the candidate gene of another second fusion gene acquired by the information processing apparatus 1. In a case where the obtained similarity is less than the threshold value for all the second fusion genes acquired by the information processing apparatus 1, the fusion gene filter 236 determines that the variant base sequence does not contain the fusion gene of the gene similar to the candidate gene of any second fusion gene.

In addition, in a case where the similarity between the base sequence of the candidate gene of the second fusion gene acquired by the information processing apparatus 1 and the base sequence of one gene of the fusion genes included in the variant base sequence is 65% or more and 100% or less, the fusion gene filter 236 may determine that the fusion gene in which the gene having the base sequence similar to the base sequence of the candidate gene of the second fusion gene and another gene are fused is included in the variant base sequence. Preferably, in a case where the similarity between the base sequence of the candidate gene of the second fusion gene and the base sequence of one gene of the fusion genes included in the variant base sequence is 80% or more and 100% or less, the fusion gene filter 236 may determine that the fusion gene in which the gene having the base sequence similar to the base sequence of the candidate gene of the second fusion gene and another gene are fused is included in the variant base sequence.

In addition, the fusion gene filter 236 may transmit the variant base sequence to an external server that stores a plurality of second fusion genes. The fusion gene filter 236 checks whether or not the variant base sequence includes a fusion gene of a gene similar to any of the plurality of candidate genes of the second fusion gene registered in a database of the external server. The fusion gene filter 236 may determine that the variant base sequence includes the gene similar to the candidate gene of the second fusion gene in a case of receiving a notification indicating that the variant base sequence includes a fusion gene of a gene similar to any of the candidate genes of the plurality of registered second fusion genes from the external server.

The fusion gene filter 236 determines a category on the basis of the determination result as to whether or not a fusion gene in which two genes respectively similar to the two candidate genes of the first fusion gene are fused is included in the variant base sequence. For example, in a case where it is determined that, for any of a plurality of first fusion genes acquired by the information processing apparatus 1, a fusion gene in which two genes respectively similar to two candidate genes of the first fusion gene are fused is included in the variant base sequence, the fusion gene filter 236 determines a category corresponding to a sequence variation to be analyzed assuming that there is a risk of being harmful (for example, "2" is subtracted from the category as the second predetermined amount), and passes the process to the structure filter 238.

As described above, the fusion gene filter 236 can accurately estimate the degree of risk of a sequence variation being harmful by using a category with reference to base sequences of two candidate genes of the first fusion gene known to have a relatively high possibility of a driver variation.

The fusion gene filter 236 determines a category on the basis of a determination result as to whether the variant base sequence includes the fusion gene in which the gene having the base sequence similar to the base sequence of the candidate gene of the second fusion gene is fused with another gene. For example, in a case where it is determined that the variant base sequence includes a gene similar to any of the candidate genes of the plurality of second fusion genes acquired by the information processing apparatus 1, the fusion gene filter 236 determines a category corresponding to the sequence variation to be analyzed assuming that there is a risk of being harmful (for example, "1" is subtracted from the category as the first predetermined amount), and passes the process to the preservation position filter 237.

In a case where it is determined that the fusion gene of the candidate genes respectively similar to the two candidate genes of the first fusion gene acquired by the information processing apparatus 1 is not included in the variant base sequence, or in a case where it is determined that the fusion gene of the gene similar to the candidate gene of the second fusion gene is not included in the variant base sequence, the fusion gene filter 236 sets the category without any change (here, since the initial category is MYC3, the category is set to MYC3 without any change) and passes the process to the preservation position filter 237.

Even in a case where one of combinations of the two candidate genes of the fusion gene is not registered in the storage unit 12, it is known that there is a possibility that the second fusion gene including a specific candidate gene will be a driver variation. By referring to the base sequence of the candidate gene of the second fusion gene, the fusion gene filter 236 can more accurately present the degree of risk of the sequence variation being harmful by using a category.

### <Preservation position filter>

A preserved sequence preserved between genomes of different species often play an important role in the physiological activity of cells. Therefore, in a case where a variation occurs at a position of the preserved sequence, the risk of sequence variation being harmful is relatively high. The preservation position filter 237 determines a category on the basis of whether or not a position of a preserved sequence that is a base sequence preserved between genomes of different species is included in a variation site of a sequence variation. Here, the preservation position filter 237 provides a threshold value based on a value indicating the degree of preservation (an output value of a preservation degree prediction tool such as GERP or phylop PhastCons), and only a preserved sequence exceeding the threshold value may be used for classification.

In a case where it is determined that the position of the preserved sequence is included in the variation site, the preservation position filter 237 determines a category corresponding to the sequence variation to be analyzed assuming that there is a risk of being harmful (for example, "1" is subtracted from the category as the first predetermined amount), and passes the process to the structure filter 238. On the other hand, in a case where it is determined that the position of the preserved sequence is not included in the variation site, the preservation position filter 237 sets the category without any change and passes the process to the structure filter 238. As described above, the preservation position filter 237 can present the degree of the risk of the sequence variation corresponding to this variation site being harmful more accurately with the category by using information indicating the position of the preserved sequence.

In addition, it is known that, in a case where structural variations such as chromosomal translocation, deletion of an important gene, and variations extending over a plurality of genes occur, a risk of these structural variations being harmful is relatively high. The structure filter 238 determines whether the sequence variation indicated by the base sequence information is a structural variation such as chromosomal translocation.

### <Structure filter>

The structure filter 238 determines whether or not a sequence variation indicated by base sequence information is chromosomal translocation, and determines a category on the basis of the determination result. The structure filter 238 refers to the content of the variation and the variation site included in the sequence variation indicated by the base sequence information, and determines whether or not chromosomal translocation has occurred. In addition, the structure filter 238 may determine whether or not the sequence variation is chromosomal translocation by dividing a variant base sequence corresponding to the sequence variation into a plurality of base sequences and specifying a position on a genome for each of the base sequences after division.

The structure filter 238 determines whether or not the sequence variation represented by the base sequence information is a variation extending over a plurality of genes, and determines a category on the basis of the determination result. The structure filter 238 refers to the content of the variation or the variation site included in any sequence variation indicated by the base sequence information, and determines whether or not the variation extending over a plurality of genes has occurred. The structure filter 238 may divide the variant base sequence corresponding to the sequence variation into a plurality of base sequences, and specify a position on the genome for each of the base sequences after division, thereby determining whether or not the sequence variation is a variation extending over a plurality of genes.

In the storage unit 12, information indicating a plurality of registered genes involved in canceration of cells and the like is registered in advance. The information indicating registered genes is, for example, identification information for identifying a registered gene or information indicating a position of a registered gene on a chromosome. The structure filter 238 may determine whether or not the sequence variation indicated by the base sequence information is deletion of the registered gene, and determine a category on the basis of the determination result. The structure filter 238 determines whether or not any of the plurality of registered genes registered in the storage unit 12 has been deleted by referring to the content of the variation or the variation site included in any sequence variation indicated by the base sequence information.

In the storage unit 12, position information on a chromosome of an enhancer that controls expression of a gene involved in canceration of a cell or the like is registered in advance. In a case where it is determined that translocation, inversion, deletion, or the like has occurred, the structure filter 238 may determine whether or not an oncogene in which the sequence variation represented by the base sequence information is registered in the storage unit 12 is in decontrol abnormality of being located near an enhancer registered in the storage unit 12, and determine the category on the basis of the determination result.

Information regarding directions (5'→3' and 3'→5') of the gene region in the genome is registered in advance in the storage unit 12. In a case where it is determined that a sequence variation represented by base sequence information forms a fusion gene such as a first fusion gene or a second fusion gene due to translocation, deletion, or the like, when two genes that form a fusion gene are defined as a first candidate gene and a second candidate gene, the structure filter 238 may determine whether directions of the first candidate gene and the second candidate gene are the same direction (for example, a combination of the 5',3' direction of the first candidate gene and the 5',3' direction of the second candidate gene, or a combination of the 3'→5' direction of the first candidate gene and the 3'→5' direction of the second candidate gene), determine whether a functional fusion gene is formed, and determine a category on the basis of the determination result.

In the storage unit 12, amino acid translation (codon) of a gene region and sequence information related to RNA splicing are registered in advance. In a case where it is determined that the sequence variation represented by the base sequence information forms a fusion gene due to translocation, deletion, or the like, the structure filter 238 may determine whether or not a functional fusion gene is formed on the basis of the information of the above item, and determine a category on the basis of the determination result.

In addition, the structure filter 238 divides the variant base sequence into a plurality of base sequences, and specifies a position on a genome for each of the base sequences after division. The structure filter 238 may determine whether or not deletion of any registered gene has occurred by comparing the position of the specified base sequence on the genome with positions of a plurality of registered genes registered in the storage unit 12.

In a case where it is determined that translocation has occurred, the structure filter 238 determines a category corresponding to the sequence variation to be analyzed assuming that there is a risk of being harmful. For example, the structure filter 238 subtracts "1" as the first predetermined amount from the category corresponding to the sequence variation. On the other hand, in a case where it is determined that no translocation has occurred, the category corresponding to the sequence variation to be analyzed is left without any change.

In a case where it is determined that a variation extending over a plurality of genes has occurred, the structure filter 238 determines a category corresponding to the sequence variation to be analyzed assuming that there is a risk of being harmful (for example, "1" as the first predetermined amount is subtracted from the category corresponding to the sequence variation). On the other hand, in a case where it is determined that no structural variation extending over a plurality of genes has occurred, the structure filter 238 leaves the category corresponding to the sequence variation without any change.

In a case where it is determined that any of the plurality of registered genes registered in the storage unit 12 is deleted, the structure filter 238 further subtracts the first predetermined amount from the category corresponding to the sequence variation to be analyzed, and passes the process to the structure filter 238. On the other hand, in a case where it is determined that none of the plurality of genes registered in the storage unit 12 is deleted, the structure filter 238 leaves the category corresponding to the sequence variation to be analyzed without any change, and passes the process to the structure filter 238. As described above, the structure filter 238 determines whether or not a structural variation such as chromosomal translocation, a variation extending over a plurality of genes, or a deletion of a gene involved in canceration of a cell or the like has occurred, and can thus more accurately present the degree of risk of a sequence variation being harmful by using a category.

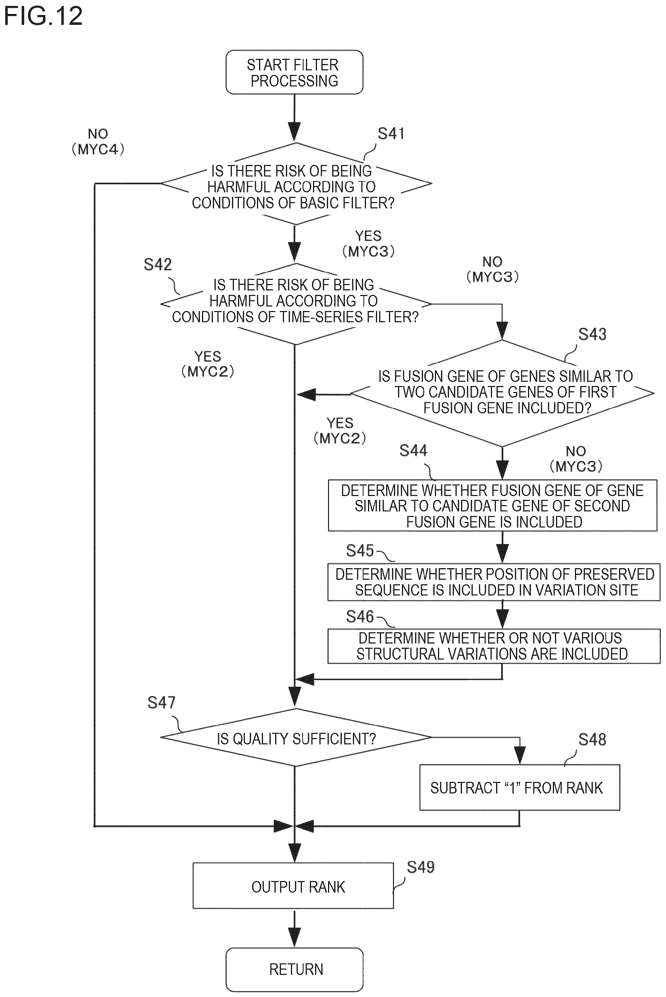

Fig. 12 is a flowchart illustrating details of a flow of the filter processing of the third filter processing unit 43 having the functional configuration in Fig. 11.

In step S41, the basic filter 231 determines whether or not there is a risk of being harmful for the sequence variation to be processed according to the conditions of the basic filter 231. In a case where the sequence variation to be processed has no risk of being harmful according to the conditions of the basic filter 231, "NO" is determined in step S41, the category is set to MYC4, and the process proceeds to step S49.

In step S49, the third filter processing unit 43 outputs the category.

In a case where the sequence variation to be processed has a risk of being harmful according to the conditions of the basic filter 231, "YES" is determined in step S41, the category is set to MYC3, and the process proceeds to step S42.

In step S42, the time-series filter 232 determines whether or not there is a risk of being harmful for the sequence variation to be processed according to the conditions of the time-series filter 232.

In a case where the sequence variation to be processed has a risk of being harmful according to the conditions of the time-series filter 232, "YES" is determined in step S42, the category is set to MYC2, and the process proceeds to step S47. Note that the processes in and after step S47 will be described later.

In a case where the sequence variation to be processed has no risk of being harmful according to the conditions of the time-series filter 232, "NO" is determined in step S42, the category is set to MYC3, and the process proceeds to step S43.

In step S43, the fusion gene filter 236 determines whether or not the sequence variation to be processed includes a fusion gene of genes similar to two candidate genes of the first fusion gene.

In a case where the sequence variation to be processed includes a fusion gene of genes similar to two candidate genes of the first fusion gene (that is, in a case where there is a risk of being harmful,), "YES" is determined in step S43, the category is set to MYC2, and the process proceeds to step S47. Note that the processes in and after step S47 will be described later.

In a case where the sequence variation to be processed does not include a fusion gene of genes similar to two candidate genes of the first fusion gene (that is, in a case where there is no risk of being harmful,), "NO" is determined in step S43, the category is set to MYC3, and the process proceeds to step S44.

In step S44, the fusion gene filter 236 determines whether the sequence variation to be processed includes a fusion gene of a gene similar to a candidate gene of the second fusion gene.

In step S45, the preservation position filter 237 determines whether a position of the preserved sequence is included in the variation site for the sequence variation to be processed.

In step S46, the structure filter 238 determines whether or not the sequence variation to be processed includes various structural variations. In each of the filters in steps S44 to S46, in a case where it is determined that there is a risk of being harmful, the category is set to MYC2. On the other hand, in a case where it is determined that there is no risk of being harmful, the category is set to MYC3.

In step S47, the quality filter 235 determines whether or not the quality is sufficient.

In a case where the quality of the results of the processes in steps S41 to S46 (Results of filter processing of basic filter 231, time-series filter 232, fusion gene filter 236, preservation position filter 237, and structure filter 238) is sufficient, "YES" is determined in step S47, and the process proceeds to step S48. In step S47, since it is determined that the quality is sufficient, "1" is subtracted from the category.

In a case where the quality of the results of the processes in steps S41 to S46 (filtering results in the basic filter 231, the time-series filter 232, the fusion gene filter 236, the preservation position filter 237, and the structure filter 238) is not sufficient, "NO" is determined in step S47, and the process proceeds to step S49. In this case, since it is determined in step S47 that the quality is not sufficient, "1" is not subtracted from the category.

In step S49, the third filter processing unit 43 outputs the category.

Hereinafter, an example of a method in which the adjustment unit 4 adjusts each filter of the third filter processing unit 43 in the second embodiment will be described. Note that examples of adjustment of the basic filter 231, the time-series filter 232, and the quality filter 235 are similar to those in the first embodiment, and thus descriptions thereof will be omitted.

### <<Example of adjustment of fusion gene filter>>

As described above, in an embodiment of the fusion gene filter 236, it is determined that the fusion gene is included in a variant base sequence in a case where the similarity between the two base sequences in which the two candidate genes of the first fusion gene are encoded and at least a part of the base sequences included in the variant base sequence is equal to or more than a threshold value in both cases. Here, the adjustment unit 4 can adjust a determination result from the fusion gene filter 236 by adjusting the threshold value.

Furthermore, as described above, in one embodiment of the fusion gene filter 236, in a case where the similarity between the base sequences of the two candidate genes of the first fusion gene acquired by the information processing apparatus 1 and the base sequences of the two genes of the fusion gene included in the variant base sequence is 65% or more and 100% or less, it can be determined that a fusion gene in which two genes respectively similar to the two candidate genes of the first fusion gene are fused is included in the variant base sequence. Here, the adjustment unit 4 can adjust the determination result from the fusion gene filter 236 by adjusting a range of the ratio of the similarity related to the determination. For example, in a case where each of the similarities between the base sequences of the two candidate genes of the first fusion gene and the base sequences of the two genes of the fusion gene included in the variant base sequence is 75% or more and 100% or less, it can be determined that the fusion gene is included in the variant base sequence, or in a case where the similarity is 85% or more and 100% or less, it can be determined that the fusion gene is included in the variant base sequence.

In addition, as described above, in one embodiment of the fusion gene filter 236, a variant base sequence corresponding to the sequence variation to be analyzed is transmitted to an external server that stores a combination of candidate genes of a plurality of first fusion genes, and it can be determined that the fusion gene is included in the variant base sequence on the basis of a check result in the external server. Here, the adjustment unit 4 can adjust the determination result from the fusion gene filter 236 by changing an external server to be used.

In addition, as described above, in one embodiment of the fusion gene filter 236, for the plurality of second fusion genes acquired by the information processing apparatus 1, the similarity between the base sequence of the candidate gene of the second fusion gene and the base sequence of one gene of the fusion genes included in the variant base sequence is obtained for each second fusion gene. In a case where the obtained similarity is equal to or more than a threshold value, the fusion gene filter 236 determines that the variant base sequence includes the fusion gene of the genes similar to the candidate genes of the second fusion gene acquired by the information processing apparatus 1. Here, the adjustment unit 4 can adjust the determination result from the fusion gene filter 236 by adjusting the threshold value of the similarity.

Furthermore, as described above, in one embodiment of the fusion gene filter 236, in a case where the similarity between the base sequence of the candidate gene of the second fusion gene acquired by the information processing apparatus 1 and the base sequence of one gene of the fusion genes included in the variant base sequence is 65% or more and 100% or less, it can be determined that a fusion gene in which a gene having a base sequence similar to the base sequence of the candidate gene of the second fusion gene and another gene are fused is included in the variant base sequence. Here, the adjustment unit 4 can adjust the determination result from the fusion gene filter 236 by adjusting a range of the ratio of the similarity related to the determination. For example, in a case where the similarity between the base sequence of the candidate gene of the second fusion gene and the base sequence of one gene of the fusion genes included in the variant base sequence is 75% or more and 100% or less, it can be determined that the fusion gene is included in the variant base sequence, or in a case where the similarity is 85% or more and 100% or less, it can be determined that the fusion gene is included in the variant base sequence.

In addition, as described above, in one embodiment of the fusion gene filter 236, the variant base sequence may be transmitted to an external server storing a plurality of second fusion genes, and it may be determined that the variant base sequence includes a gene similar to the candidate gene of the second fusion gene on the basis of a check result in the external server. Here, the adjustment unit 4 can adjust the determination result from the fusion gene filter 236 by changing an external server to be used.

### <<Example of adjustment of preservation position filter>>

The preservation position filter 237 determines whether or not a position of the preserved sequence indicated by the preserved sequence position information acquired by the information processing apparatus 1 is included in the variation site, but the classification criterion of or the determination result from the preservation position filter 237 can be adjusted by changing a threshold value set for use in determining whether or not the sequence is preserved.

### <<Adjustment example of structure filter>>

The structure filter 238 refers to the content of the variation or the variation site included in the sequence variation indicated by the base sequence information to determine whether or not a structural polymorphism (for example, translocation, missing, or insertion) of the chromosome has occurred, and the adjustment unit 4 can adjust the determination result from the structure filter 238 by changing the content of the variation or the variation site to be referred to. In addition, the structure filter 238 may determine whether or not the sequence variation is chromosomal translocation by dividing a variant base sequence corresponding to the sequence variation into a plurality of base sequences and specifying a position on a genome for each of the base sequences after division. On the other hand, the adjustment unit 4 can adjust the determination result from the structure filter 238 by changing the unit of division.

In addition, in one embodiment of the structure filter 238, in a case where it is determined that translocation, inversion, deletion, or the like has occurred, it may be determined whether or not the sequence variation represented by the base sequence information is in decontrol abnormality of being located near an enhancer of a cancer gene, and the category may be determined on the basis of the determination result. Here, the adjustment unit 4 can adjust the determination result by adjusting the criteria by which the structure filter 238 determines that the decontrol abnormality has occurred.

Although one embodiment of the present invention has been described above, the present invention is not limited to the above-described embodiment, and modifications, improvements, and the like within the scope of achieving the object of the present invention are considered to be included in the present invention.

Furthermore, the system configuration illustrated in Fig. 1 and the configuration of the controlling unit 11 of the information processing apparatus 1 illustrated in Fig. 2 are merely examples for achieving the object of the present invention, and are not particularly limited.

The functional block diagrams illustrated in Figs. 2, 3, 5, 7, and 11 are merely examples, and are not particularly limited. That is, it is sufficient that the information processing apparatus 1 has a function capable of executing the above-described series of processes as a whole, and what functional block is used to realize this function is not particularly limited to the examples in these drawings.

In addition, a location of the functional block is not limited to Figs. 2, 3, 5, 7, and 11, and may be any location. For example, in the example in Fig. 2, the above-described processes are performed on the information processing apparatus 1 side, but the present invention is not limited thereto, and at least some of the processes may be performed on another information processing apparatus side (not illustrated). In other words, the information processing apparatus 1 has a configuration including functional blocks necessary for execution of the analysis process, but this is merely an example. At least some of the functional blocks disposed on the information processing apparatus 1 side may be provided on another information processing apparatus side (not illustrated).

Means and methods for performing various processes in the system according to the above-described embodiment can be realized by either a dedicated hardware circuit or a programmed computer. The program may be provided by, for example, computer-readable recording media such as a flexible disk and a CD-ROM, or may be provided online via a network such as the Internet. In this case, the program recorded on the computer-readable recording medium is normally transferred to and stored in the storage unit 12 such as a hard disk. In addition, the program may be provided as independent application software, or may be incorporated into software of a device as one function of a system.

Note that, in the present specification, steps for describing the program recorded on the recording medium include not only processes executed in chronological order according to the order, but also processes executed in parallel or individually without necessarily being executed in chronological order.

In addition, one embodiment of the present invention includes a standard nucleic acid composition that contains a nucleic acid including a sequence variation of which a category to which the sequence variation is to belong is known and is used in the information processing apparatus 1 described above. In addition, standard nucleic acid data that includes a sequence variation of which a category to which the sequence variation is to belong is known and is used in the information processing apparatus 1 described above is also included.

Furthermore, in the present specification, the term of the system indicates an overall device including a plurality of devices, a plurality of means, and the like.

The present invention includes the following aspects and forms.

[1] An information processing apparatus that selects a target sequence variation possessed by a subject and having a risk of being harmful, the information processing apparatus including:
   a filtering unit that classifies one or more sequence variations specified by sequencing a nucleic acid included in the subject into different categories according to a degree of risk of being harmful on the basis of one or more classification criteria; and
   a control unit that classifies a base sequence including a sequence variation of which a category to which the sequence variation is to belong is known into each of the categories according to the degree of risk of being harmful on the basis of at least one of the classification criteria, and compares a classification result with the category to which the sequence variation is to belong.
[2] The information processing apparatus according to [1], further including an adjustment unit that adjusts the classification criterion and/or the classification result in the filtering unit on the basis of a comparison result in the control unit.
[3] The information processing apparatus according to [1] or [2], in which the base sequence including the sequence variation of which the category to which the sequence variation is to belong is known includes two or more types of sequence variations whose categories to which the sequence variations are to belong are different from each other.
[4] The information processing apparatus according to [3], in which the base sequence including the two or more types of sequence variation whose categories to which the sequence variations are to belong are different from each other include a sequence variation that causes a specific disease and a base sequence that does not cause the specific disease.
[5] The information processing apparatus according to [4], in which the target sequence variation is a driver variation of the specific disease, and
   the two or more types of the sequence variations include a sequence variation that causes the specific disease and a sequence variation that does not cause the specific disease.
[6] The information processing apparatus according to any one of [1] to [5], in which the classification criterion is able to be changed or selected.
[7] The information processing apparatus according to [6], in which the filtering unit and the control unit are executed after the classification criterion is changed or selected.
[8] The information processing apparatus according to any one of [1] to [7], in which the base sequence to be classified by the control unit is obtained by sequencing a standard composition of nucleic acids including a sequence variation of which a category to which the sequence variation is to belong is known.
[9] The information processing apparatus according to [8], in which a condition for sequencing the standard composition and a condition for sequencing the nucleic acid included in the subject are the same.
[10] An information processing method, which is a method of selecting a target sequence variation possessed by a subject and having a risk of being harmful, the method including:
   a filtering step of classifying one or more sequence variations specified by sequencing a nucleic acid included in the subject into different categories according to a degree of risk of being harmful on the basis of one or more classification criteria; and
   a control step of classifying a base sequence including a sequence variation of which a category to which the sequence variation is to belong is known into each of the categories according to the degree of risk of being harmful on the basis of at least one of the classification criteria, and comparing a classification result with the category to which the sequence variation is to belong.
[11] An information processing program for causing a computer to function as the information processing apparatus according to any one of [1] to [9].
[12] A standard nucleic acid composition containing a nucleic acid including a sequence variation of which a category to which the sequence variation is to belong is known, the standard nucleic acid composition being used in the information processing apparatus according to any one of [1] to [9].
[13] Standard nucleic acid data including a sequence variation of which a category to which the sequence variation is to belong is known, the standard nucleic acid data being used in the information processing apparatus according to any one of [1] to [9].

### Industrial Applicability

The information processing apparatus of the present invention can present a more accurate analysis result in an apparatus that performs analysis of a possibility of a variation of a base sequence affecting the occurrence or progression of a disease, and can thus be applied to a wide range of fields such as the medical field and the life science field, and is industrially useful.

### Reference Signs List

1: information processing apparatus
2: filtering unit
3: control unit
4: adjustment unit
11: controlling unit
12: storage unit
13: communication unit
14: display unit
15: operation receiving unit
16: drive
17: removable medium
18: bus
21: first data reception unit
22: first setting reception unit
23: first filter processing unit
24: category determination unit
25: analysis result output unit
31: second data reception unit
32: second setting reception unit
33: second filter processing unit
34: comparison unit
35: comparison result output unit
43: third filter processing unit
231: basic filter
232: time-series filter
233: database filter
234: function prediction filter
235: quality filter
236: fusion gene filter
237: preservation position filter
238: structure filter

## Claims

1. An information processing apparatus that selects a target sequence variation possessed by a subject and having a risk of being harmful, the information processing apparatus comprising:
a filtering unit that classifies one or more sequence variations specified by sequencing a nucleic acid included in the subject into different categories according to a degree of risk of being harmful on the basis of one or more classification criteria; and
a control unit that classifies a base sequence including a sequence variation of which a category to which the sequence variation is to belong is known into each of the categories according to the degree of risk of being harmful on the basis of at least one of the classification criteria, and compares a classification result with the category to which the sequence variation is to belong.

2. The information processing apparatus according to claim 1, further comprising an adjustment unit that adjusts the classification criterion and/or the classification result in the filtering unit on the basis of a comparison result in the control unit.

3. The information processing apparatus according to claim 1 or 2, wherein the base sequence including the sequence variation of which the category to which the sequence variation is to belong is known includes two or more types of sequence variations whose categories to which the sequence variations are to belong are different from each other.

4. The information processing apparatus according to claim 3, wherein the base sequence including the two or more types of sequence variations whose categories to which the sequence variations are to belong are different from each other include a sequence variation that causes a specific disease and a base sequence that does not cause the specific disease.

5. The information processing apparatus according to claim 4, wherein
the target sequence variation is a driver variation of the specific disease, and
the two or more types of the sequence variations include a sequence variation that causes the specific disease and a sequence variation that does not cause the specific disease.

6. The information processing apparatus according to any one of claims 1 to 5, wherein the classification criterion is able to be changed or selected.

7. The information processing apparatus according to claim 6, wherein the filtering unit and the control unit are executed after the classification criterion is changed or selected.

8. The information processing apparatus according to any one of claims 1 to 7, wherein the base sequence to be classified by the control unit is obtained by sequencing a standard composition of nucleic acids including a sequence variation of which a category to which the sequence variation is to belong is known.

9. The information processing apparatus according to claim 8, wherein a condition for sequencing the standard composition and a condition for sequencing the nucleic acid included in the subject are the same.

10. An information processing method, which is a method of selecting a target sequence variation possessed by a subject and having a risk of being harmful, the method comprising:
a filtering step of classifying one or more sequence variations specified by sequencing a nucleic acid included in the subject into different categories according to a degree of risk of being harmful on the basis of one or more classification criteria; and
a control step of classifying a base sequence including a sequence variation of which a category to which the sequence variation is to belong is known into each of the categories according to the degree of risk of being harmful on the basis of at least one of the classification criteria, and comparing a classification result with the category to which the sequence variation is to belong.

11. An information processing program for causing a computer to function as the information processing apparatus according to any one of claims 1 to 9.

12. A standard nucleic acid composition containing a nucleic acid including a sequence variation of which a category to which the sequence variation is to belong is known, the standard nucleic acid composition being used in the information processing apparatus according to any one of claims 1 to 9.

13. Standard nucleic acid data comprising a sequence variation of which a category to which the sequence variation is to belong is known, the standard nucleic acid data being used in the information processing apparatus according to any one of claims 1 to 9.
